# EUROPÄISCHE PATENTSCHRIFT

(11) **EP 2 227 498 B1**
(45) Veröffentlichungstag und Bekanntmachung des Hinweises auf die Patenterteilung: **29.06.2011**
(21) Anmeldenummer: 08868473.3
(22) Anmeldetag: 16.12.2008
(51) Int. Cl.: C08F 220/58

(54) **WASSERLÖSLICHE ODER WASSERQUELLBARE POLYMERE AUF BASIS VON SALZEN DER ACRYLOYLDIMETHYLTAURINSÄURE ODER IHRER DERIVATE, DEREN HERSTELLUNG UN DEREN VERWENDUNG ALS VERDICKER, STABILISATOR UND KONSISTENZGEBER**
WATER-SOLUBLE OR WATER-SWELLABLE POLYMERS ON THE BASIS OF SALTS OF ACRYLOYLDIMETHYLTAURINE ACID OR THE DERIVATIVES THEREOF, THE PRODUCTION THEREOF AND THE USE THEREOF AS THICKENER, STABILIZER AND CONSISTENCY AGENTS
POLYMÈRES SOLUBLES DANS L'EAU OU POUVANT GONFLER DANS L'EAU À BASE DE SELS D'ACIDE D'ACRYLOYLDIMÉTHYLTAURINE OU DE LEURS DÉRIVÉS, LEUR FABRICATION ET LEUR UTILISATION COMME ÉPAISSISSANT, STABILISANT ET AGENT DE CONSISTANCE

(30) Priorität: 21.12.2007 DE 102007061969
(43) Veröffentlichungstag der Anmeldung: 15.09.2010
(73) Patentinhaber: Clariant Finance (BVI) Limited, Road Town, Tortola (VG)
(72) Erfinder: LOEFFLER, Matthias, 65510 Idstein (DE); LINDNER, Thomas, 68199 Mannheim (DE); BACK, Ute, 63825 Blankenbach (DE); HORNUNG, Michael, 65926 Frankfurt am Main (DE)
(74) Vertreter: Paczkowski, Marcus
(86) Internationale Anmeldenummer: PCT/EP2008/010677
(87) Internationale Veröffentlichungsnummer: WO 2009/083130

(56) Entgegenhaltungen:
- EP-A1- 0 815 828
- EP-A1- 1 033 378
- EP-A1- 1 116 733
- EP-A1- 1 251 142
- DE-A1- 19 625 810

## Beschreibung

Die vorliegende Erfindung betrifft
- vernetzte oder unvernetzte wasserlösliche oder wasserquellbare Polymere oder Polymerisate auf Basis von Acryl-, Methacryl- oder Ethacrylamidoalkylsulfonsäuresalzen, wobei die Gegenionen der genannten Sulfonsäuren Mischungen aus Alkylammonium und anderen Kationen ausgewählt aus NH₄⁺, Li⁺, Na⁺, K⁺, Ca⁺⁺, Mg⁺⁺, Zn⁺⁺ und Al⁺⁺⁺ und gegebenenfalls zusätzlich H⁺ darstellen, insbesondere deren Copolymerisate mit cyclischen N-Vinylcarbonsäureamiden oder cyclischen und linearen N-Vinylcarbonsäureamiden,
- ein Verfahren zu deren Herstellung,
- deren Verwendung als Verdicker, Konsistenzgeber und Stabilisator, insbesondere deren Verwendung als Verdicker und Konsistenzgeber von wässrigen Systemen und Systemen mit einem hohen Anteil an Ölkomponente und als Stabilisator von Emulsionen und Dispersionen, insbesondere von kosmetischen, dermatologischen und pharmazeutischen Zusammensetzungen sowie
- kosmetische, dermatologische und pharmazeutische Zusammensetzungen enthaltend ein oder mehrere der oben genannten Polymere.

Wasser- oder lösungsmittelhaltige Mehrkomponentensysteme wie Lösungen, Emulsionen oder Suspensionen werden häufig aus ökonomischen oder anwendungstechnischen Gründen oder aus Stabilitätsgründen auf höhere Viskositäten eingestellt bzw. verdickt. So kann z. B. durch Erhöhung der Viskosität der externen oder internen Phase von Emulsionen oder Suspensionen erreicht werden, dass die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit, insbesondere auf unebenen Flächen, verbessert. Dies gilt insbesondere für Hautpflegemittel und pharmazeutische Salben auf der Haut. Bei vielen technischen Produkten wie Tapetenablösern, Abbeizmitteln oder Flugzeugenteisem verhindert die erhöhte Viskosität ein vorzeitiges Abfließen von der zu behandelnden Fläche. Durch die gleichmäßigere Verteilung und verlängerte Einwirkdauer wird so die Wirksamkeit erhöht. Neben den erwähnten anwendungstechnischen Vorteilen bietet die hohe Viskosität solcher Präparate auch weitere Vorteile bei der Herstellung, Verpackung, Abfüllung und Lagerung sowie beim Transport, insbesondere ist hier aus sicherheitstechnischer Hinsicht die Verdickung saurer Medien von Bedeutung. Generell sind die rheologischen Eigenschaften bei der Herstellung und/oder Formulierung kosmetischer, dermatologischer, pharmazeutischer oder technischer Präparate ein entscheidendes Kriterium für den Einsatz dieser Produkte in der Praxis. Die eingesetzten Verdicker sollen dabei bereits in möglichst geringen Einsatzmengen zu einer ausreichenden Verdickung führen. Jedoch sollen die Farbe und prinzipiellen Eigenschaften des zu verdickenden Mediums nicht verändert werden.

Kosmetische Hautpflegemittel wie Cremes und Lotionen liegen meistens in Form von Öl-in-Wasser-Emulsionen oder Wasser-in-Öl-Emulsionen vor.

Öl-in-Wasser-Emulsionen bestehen aus einer internen Ölphase und einer externen, kontinuierlichen Wasserphase, die zur Stabilisierung der Formulierung generell mit Hilfe von Polymeren verdickt wird. Diese Emulsionen verleihen der Haut beim Auftragen ein weiches, pflegendes und angenehmes Gefühl.

Die Emulsionen werden im Allgemeinen durch Einarbeitung emulgierender Tenside vom Öl-in-Wasser-Typ (O/W) oder vom Wasser-in-Öl-Typ (W/O) zusätzlich stabilisiert. Um eine hinreichende Stabilität der Emulsionen zu erreichen, müssen derartige Tenside jedoch meist in Mengen bis zu 10 Gew.-%, bezogen auf das Gesamtgewicht der Emulsionen, zuzugeben werden. Emulsionen ohne Tenside zeigen im Allgemeinen eine unzureichende Stabilisierung der Ölkomponenten, was zur Koagulation und Separation der Ölphasen führt.

Ein Ziel der Hautpflege ist es, den durch tägliches Waschen verursachten Fett- und Wasserverlust der Haut auszugleichen, die Haut vor Umwelteinflüssen, insbesondere vor Sonne und Wind zu schützen, eine Barriere gegen Schmutz, Chemikalien und Mikroorganismen zu schaffen, dem Verlust von körpereigenen Stoffen (z. B. Wasser, natürliche Fette, Elektrolyte) entgegenzuwirken oder auszugleichen und die Hautalterung zu verzögern. Für viele Anwendungen, z. B. in der Babypflege, ist es daher hilfreich, kosmetische Hautpflegemittel mit sehr hohem Anteil an Öl- und Hautpflegekomponenten anzubieten. Unter "sehr hohem Ölanteil" wird hier ein Anteil von 35 Gew.-% und mehr verstanden. Die Stabilisierung von sehr hohem Ölanteil stellt eine zusätzliche Herausforderung dar, oft werden entsprechend hohe Mengen an Emulgatoren und Stabilisatoren eingesetzt.

Nachteilig am Einsatz der Emulgatoren ist, dass sie zu einer Reizung der Haut, den Augen und der Kopfhaut führen können oder im Einzelfall sogar eine allergische Reaktion auslösen können. So ist z. B. bekannt, dass bestimmte Emulgatoren bei gleichzeitiger Exposition von Sonnenlicht Lichtdermatosen auslösen können.
Ein weiterer Nachteil ist, dass hohe Emulgatorkonzentrationen zu einem rauen, klebrigen oder zähen Gefühl der Mittel führen können oder die Mittel kompakt und schwer erscheinen lassen. Darüber hinaus müssen die Emulgatoren in Abhängigkeit von der Polarität der Öle ausgewählt werden, so dass die Vielfalt der Formulierungen beschränkt ist.

Die Anwender von Emulsionen sind daher ständig bemüht, den Emulgatorgehalt zu reduzieren, um die Verträglichkeit der Emulsionen zu verbessern und ihre kosmetischen Eigenschaften zu optimieren.

Im Laufe der letzten Jahre etablierten sich Polymere auf dem Markt, die die Formulierung von emulgatorarmen oder sogar emulgatorfreien Emulsionen ermöglichen (WO 96/37180 und US 5,736,125). Bei den Polymeren handelt es sich um hydrophobe Modifikationen konventioneller Poly(meth)acrylate, die sowohl verdickende als auch emulgierende/dispergierende Eigenschaften aufweisen. Beispiele für kommerzielle Produkte sind Pemulen^{®} TR-1 und TR-2 von Noveon und Aculyn^{®} 22 und Aculyn^{®} 28 von Rohm & Haas.

Da derart hydrophob modifizierte Polymere ausnahmslos auf der Basis von (Meth)acrylsäure aufgebaut sind, besitzen sie folglich die Nachteile der Poly(meth)acrylate. Ein wesentlicher Nachteil von Verdickern auf Basis von Poly(meth)acrylsäure ist die starke pH-Abhängigkeit der Verdickungsleistung. So wird im Allgemeinen eine hinreichende Viskosität nur dann aufgebaut, wenn der pH-Wert der Formulierung oberhalb von pH 6,0 eingestellt ist und somit die Poly(meth)acrylsäure in neutralisierter Form vorliegt.

In DE 44 25 268 werden emulgatorfreie, feindisperse Öl-in-Wasser-Zubereitungen beschrieben, die Acrylsäurepolymere als Verdicker enthalten, die jedoch ebenfalls für saure Formulierungen ungeeignet sind und auch keine größeren Anteile an Ölkomponente stabilisieren können.

In EP-A-0 816 403 und WO 98/00094 sind vernetzte Homopolymere aus 2-Acrylamido-2-methyl-propan-sulfonaten und deren Verwendung als Verdicker beschrieben. EP-A-0 510 246 beschreibt vernetzte Copolymere aus N-Vinylcarbonsäureamiden und ungesättigten, mit einer Sulfonatgruppe substituierten Alkylamiden, die ebenfalls als Verdicker geeignet sind. In US 5,080,809 sind unververnetzte Copolymerisate aus N-Vinylpyrrolidon und 2-Acrylamido-2-methyl-propan-sulfonat beschrieben. Diese Polymere sind jedoch ungeeignet, größere Anteile an Öl ohne Zugabe von zusätzlichen Emulgatoren zu stabilisieren.

In EP 1 116 733 werden wasserlösliche oder wasserquellbare Copolymerisate auf Basis von Ammoniumsalzen von Acrylamido-alkylsulfonsäuren und cyclischen N-Vinylcarbonsäureamiden oder cyclischen und linearen N-Vinylcarbonsäureamiden, deren Herstellung und deren Verwendung als Verdicker, Stabilisator von Emulsionen und Dispersionen offenbart. Die in EP 1 116 733 beschriebenen Polymere sind jedoch ebenfalls ungeeignet, größere Anteile an Öl ohne Zugabe von zusätzlichen Emulgatoren zu stabilisieren.

In DE 100 65 047 und DE 100 65 046 werden kosmetische oder dermatologische Gelcremes vom Typ Öl-in-Wasser, bzw. Emulsionen vom Typ Öl-in-Wasser, enthaltend Ammoniumacryloyldimethyltaurate/Vinylpyrrolidoncopolymere beschrieben. Diese Gelcremes enthalten keine größeren Anteile an Öl. Auch hier ist es erforderlich, zur Stabilisierung und Verdickung von Zusammensetzungen mit größeren Anteilen an Öl, zusätzliche Emulgatoren einzusetzen.

Aufgabe war es daher, Substanzen bereitzustellen, die in vorteilhafter Weise zur Herstellung von Zusammensetzungen, beispielsweise von kosmetischen, dermatologischen oder pharmazeutischen Zusammensetzungen, verwendet werden können, und dadurch Zusammensetzungen erhalten werden können, die auch bei einem hohen Anteil an Ölkomponenten und bei niedrigem pH-Wert sehr gute rheologische Eigenschaften zeigen, zugleich hautfreundlich und auch ohne Verwendung von Emulgatoren oder geringem Emulgatoreintrag phasenstabil sind.

Überraschenderweise wurde nun gefunden, dass diese Aufgabe durch vernetzte und unvernetzte wasserlösliche oder wasserquellbare Polymere oder Polymerisate auf Basis von Acryl-, Methacryl- und/oder Ethacrylamidoalkylsulfonsäuren, wobei die Gegenionen der genannten Sulfonsäuren Mischungen aus Alkylammonium und anderen Kationen ausgewählt aus NH₄⁺, Li⁺, Na⁺, K⁺, Ca⁺⁺, Mg⁺⁺, Zn⁺⁺ und Al⁺⁺⁺ und gegebenenfalls zusätzlich H⁺ darstellen, und insbesondere der entsprechenden Copolymerisate mit cyclischen N-Vinylcarbonsäureamiden oder cyclischen und linearen N-Vinylcarbonsäureamiden, gelöst wird.

Gegenstand der Erfindung sind Polymere enthaltend
a) eine oder mehrere der wiederkehrenden Struktureinheiten der Formel (1) 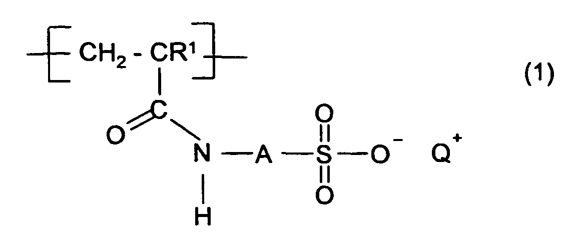 worin R¹ Wasserstoff, Methyl oder Ethyl und A C₁-C₈-Alkylen bedeutet, und Q⁺ für H⁺, NH₄⁺, Li⁺, Na⁺, K⁺, ½ C⁺⁺, ½ Mg⁺⁺, ½ Zn⁺⁺ oder ⅓ Al⁺⁺⁺ steht, und der Neutralisationsgrad der Struktureinheiten der Formel (1) von 50 bis 100 mol-%, vorzugsweise von 80 bis 100 mol-%, besonders bevorzugt von 90 bis 100 mol-% und insbesondere bevorzugt von 95 bis 100 mol-% beträgt,
   und
b) eine oder mehrere der wiederkehrenden Struktureinheiten der Formel (2) 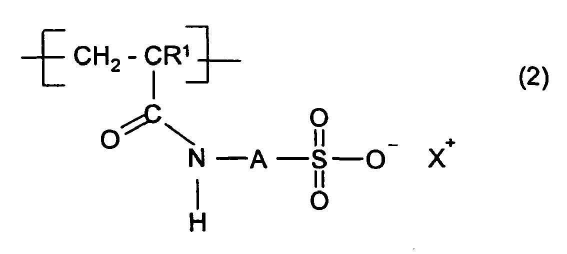 worin R¹ und A die Bedeutung von R¹ und A aus Formel (1) haben und X⁺ für [HNR⁵R⁶R⁷]⁺ steht, wobei R⁵, R⁶ und R⁷ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, mit der Maßgabe, dass das molare Verhältnis der Struktureinheiten der Formel (1), worin Q⁺ NHa⁺, Li⁺, Na⁺, K⁺, Ca⁺⁺, Mg⁺⁺, Zn⁺⁺ oder Al⁺⁺⁺ bedeutet, zu den Struktureinheiten der Formel (2) von 97 : 3 bis 55 : 45 beträgt, und die korrespondierenden Alkylammoniumchloride XCI eine kritische Mizellbildungskonzentration (critical micelle concentration, CMC) < 15 g/l besitzen,
   und
d) 0 bis 8, vorzugsweise 0,01 bis 5 Gew.-% an vernetzenden Struktureinheiten, die aus Monomeren mit mindestens zwei olefinischen Doppelbindungen hervorgegangen sind.

Im Rahmen der vorliegenden Erfindung werden die Kationen [HNR⁵R⁶R⁷]⁺ als "Alkylammonium" bezeichnet. Die Verbindungen NR⁵R⁶R⁷ werden im Rahmen der vorliegenden Erfindung als "Alkylamin" bezeichnet.

Der Neutralisationsgrad der Struktureinheiten der Formel (1) von x mol% bedeutet, dass in x mol-% der Struktureinheiten der Formel (1) Q⁺ eine andere Bedeutung als H⁺ besitzt.

Die CMC ist die Konzentration, bei der Agglomerate (Mizellen) gebildet werden. Bei Überschreitung der CMC - ab einem sog. Phasenübergangspunkt - ändert sich der Verlauf der physikalischen Eigenschaften der Lösung mit der Konzentration, insbesondere auch die Oberflächen- oder Grenzflächenspannung des Systems. Die CMC wird durch Auftragung von Grenzflächenspannung gegen Logarithmus der Konzentration ermittelt.

Die erfindungsgemäßen Polymere sind u. a. hervorragend geeignet als Verdicker und Konsistenzgeber von wässrigen Systemen, als Stabilisator von Emulsionen und Dispersionen, insbesondere von kosmetischen, dermatologischen und pharmazeutischen Zusammensetzungen mit hohem Ölanteil. Sie ergeben in Öl-in-Wasser-Emulsionen mit hohem Ölanteil auch ohne Zugabe von Tensiden oder Emulgatoren stabile Emulsionen. Vorteilhafterweise zeigen sie auch über einen weiten pH-Bereich, d. h. auch bei stark sauren pH-Werten, sehr gute Verdickungseigenschaften.

Eine bevorzugte Ausführungsform der Erfindung sind Polymere enthaltend
ab) 49,99 bis 98,99 Gew.-% einer Mischung der wiederkehrenden Struktureinheiten der Formeln (1) und (2),
c1) 1 bis 50 Gew.-% der wiederkehrenden Struktureinheit der Formel (3) 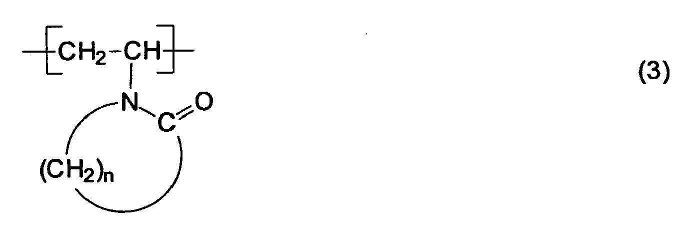 wobei n eine ganze Zahl von 2 bis 9 bedeutet,
   oder
c2) 1 bis 50 Gew.-% einer Mischung der wiederkehrenden Struktureinheit der Formel (3) und der wiederkehrenden Struktureinheit der Formel (4) 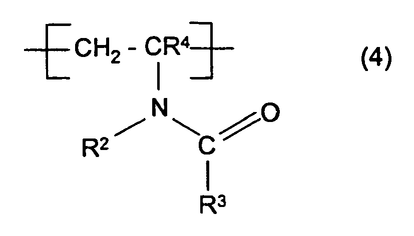 wobei R² und R³ gleich oder verschieden sein können und Wasserstoff oder eine lineare oder verzweigte Alkylgruppe mit 1 bis 22 Kohlenstoffatomen oder eine lineare oder verzweigte, einfach oder mehrfach ungesättigte Alkenylgruppe mit 2 bis 22 Kohlenstoffatomen bedeuten und R⁴ für Wasserstoff, Methyl oder Ethyl steht
   und
d) 0 bis 8, vorzugsweise 0,01 bis 5 Gew.-% an vernetzenden Struktureinheiten, die aus Monomeren mit mindestens zwei olefinischen Doppelbindungen hervorgegangen sind.

Eine weitere bevorzugte Ausführungsform der Erfindung sind Polymere enthaltend
ab) 92 bis 99,99 Gew.-% einer Mischung der wiederkehrenden Struktureinheiten der Formeln (1) und (2) und
d) 8 bis 0,01 Gew.-% an vernetzenden Struktureinheiten, die aus Monomeren mit mindestens zwei olefinischen Doppelbindungen hervorgegangen sind.

Weiterhin bevorzugte erfindungsgemäße Polymere enthalten 69,5 bis 97,5, vorzugsweise 84,5 bis 96,5 Gew.-% einer Mischung der Struktureinheiten der Formeln (1) und (2), vorzugsweise abgeleitet von der 2-Acrylamido-2-methyl-propan-sulfonsäure, 2 bis 30, vorzugsweise 3 bis 15 Gew.-% der Struktureinheiten der Formel (3) oder einer Mischung der Struktureinheiten der Formeln (3) und (4), wobei die Struktureinheiten der Formel (3) vorzugsweise abgeleitet sind von N-Vinylpyrrolidon, und 0,2 bis 3, vorzugsweise 0,5 bis 2 Gew.-% an vernetzenden Struktureinheiten, die aus Monomeren mit mindestens zwei olefinischen Doppelbindungen hervorgegangen sind. Unter diesen Polymeren sind wiederum diejenigen bevorzugt, worin zwar Struktureinheiten der Formel (3), aber keine Struktureinheiten der Formel (4), enthalten sind.

In einer besonders bevorzugten Ausführungsform der Erfindung bestehen die erfindungsgemäßen Polymere aus 69,5 bis 97,5 Gew.-% einer Mischung der Struktureinheiten der Formeln (1) und (2), vorzugsweise abgeleitet von der 2-Acrylamido-2-methyl-propan-sulfonsäure, 2 bis 30 Gew.-% der Struktureinheiten der Formel (3), vorzugsweise abgeleitet von N-Vinylpyrrolidon, und 0,5 bis 3 Gew.-% an vernetzenden Struktureinheiten, die aus Monomeren mit mindestens zwei olefinischen Doppelbindungen hervorgegangen sind.

In einer insbesondere bevorzugten Ausführungsform der Erfindung bestehen die erfindungsgemäßen Polymere aus 84,5 bis 96,5 Gew.-% einer Mischung der Struktureinheiten der Formeln (1) und (2), vorzugsweise abgeleitet von der 2-Acrylamido-2-methyl-propan-sulfonsäure, 3 bis 15 Gew.-% der Struktureinheiten der Formel (3), vorzugsweise abgeleitet von N-Vinylpyrrolidon, und 0,5 bis 2 Gew.-% an vernetzenden Struktureinheiten, die aus Monomeren mit mindestens zwei olefinischen Doppelbindungen hervorgegangen sind.

In den Struktureinheiten der Formeln (1) und (2) der erfindungsgemäßen Polymere ist R¹ vorzugsweise Wasserstoff oder Methyl und besonders bevorzugt Wasserstoff.

In den Struktureinheiten der Formeln (1) und (2) der erfindungsgemäßen Polymere ist A vorzugsweise eine Struktureinheit der Formel -CH₂-C(CH₃)₂-.

Insbesondere bevorzugt sind die Struktureinheiten der Formeln (1) und (2) der erfindungsgemäßen Polymere abgeleitet von der 2-Acrylamido-2-methyl-propan-sulfonsäure.

In den Struktureinheiten der Formel (3) der erfindungsgemäßen Polymere ist n vorzugsweise 3, d. h. die Struktureinheiten der Formel (3) sind vorzugsweise abgeleitet von N-Vinylpyrrolidon.

In den Struktureinheiten der Formel (4) der erfindungsgemäßen Polymere sind R² und R³ vorzugsweise Wasserstoff oder Methyl und R⁴ ist vorzugsweise Wasserstoff.

In den Struktureinheiten der Formel (1) der erfindungsgemäßen Polymere ist das von H⁺ verschiedene Gegenion Q⁺ vorzugsweise ausgewählt aus NH₄⁺, Alkali⁺, wobei unter Alkali⁺ wiederum Na⁺ bevorzugt ist, und Erdalkali⁺⁺. Besonders bevorzugt ist das von H⁺ verschiedene Gegenion Q⁺ NH₄⁺.

In den Struktureinheiten der Formel (2) der erfindungsgemäßen Polymere ist das Kation X⁺ vorzugsweise ausgewählt aus Laurylamidopropyldimethylammonium, Stearylamidopropyldimethylammonium, Behenylamidopropyldimethylammonium, C₁₂₋₁₈-Alkyldimethylammonium und C₂₀₋₂₂-Alkyldimethylammonium.

Die vernetzenden Strukturen der erfindungsgemäßen Polymere, die aus Monomeren mit mindestens zwei olefinischen Doppelbindungen hervorgegangen sind, leiten sich vorzugsweise ab von Acryl-, Methacryl- oder Ethacrylsäureallylester, insbesondere von Acryl- oder Methacrylsäureallylester; von Dipropylenglykol-diallylether, Polyglykoldiallylether, Triethylenglykoldivinylether, Hydrochinon-diallylether, Tetraallyloxyethan oder anderen Allyl- oder Vinylethern multifunktioneller Alkohole, Tetraethylenglykol-diacrylat, Triallylamin, Trimethylolpropandiallylether, Trimethylolpropantriacrylat, Methylenbis-acrylamid oder Divinylbenzol.

Besonders bevorzugt leiten sich die vernetzenden Strukturen der erfindungsgemäßen Polymere ab von Monomeren der allgemeinen Formel (5), 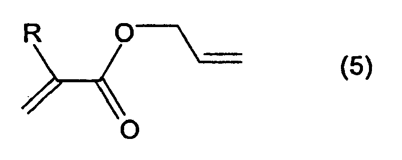 worin R Wasserstoff, Methyl oder Ethyl bedeutet.
Weiterhin besonders bevorzugt als Vernetzer für die erfindungsgemäßen Polymere ist Trimethylolpropantriacrylat (TMPTA) und Methylenbisacrylamid (MBA). Insbesondere bevorzugt ist Trimethylolpropantriacrylat.

In einer weiteren bevorzugten Ausführungsform der Erfindung beträgt die Einsatzmenge des Vernetzers zur Herstellung der erfindungsgemäßen Polymere, bezogen auf die Gesamtmasse der bei der Polymerisation zu polymerisierenden Monomere, 0,01 bis 8, bevorzugt 0,51 bis 5, besonders bevorzugt 1 bis 2,5 und insbesonders bevorzugt 1,2 bis 2 Gew.%.

Die Verteilung der verschiedenen Struktureinheiten in den erfindungsgemäßen Polymeren kann statistisch, blockartig, alternierend oder gradientenartig sein.

Die erfindungsgemäßen Polymere besitzen bevorzugt ein Molekulargewicht von 10³ bis 10⁹ g/mol, besonders bevorzugt von 10⁴ bis 10⁷ g/mol und insbesondere bevorzugt von 5*10⁵ bis 5*10⁶ g/mol.

Die Herstellung der erfindungsgemäßen Polymere erfolgt z. B. in der Weise, dass die den wiederkehrenden Struktureinheiten der Formeln (1) und (2) oder (1), (2), (3) und gegebenenfalls (4) entsprechenden Monomere in einem protischen Lösungsmittel, bevorzugt in tert.-Butanol, gelöst oder dispergiert werden, mit einer Mischung aus Ammoniak und/oder einer Li⁺-, Na⁺-, K⁺-, Ca⁺⁺-, Mg⁺⁺-, Zn⁺⁺- oder Al⁺⁺⁺-enthaltenden Base, vorzugsweise der entsprechenden Hydroxide oder Carbonate, besonders bevorzugt der Hydroxide, und Alkylamin im erfindungsgemäßen Mischungsverhältnis neutralisiert werden, zu dieser Lösung oder Dispersion gegebenenfalls ein oder mehrere Vernetzer mit mindestens zwei olefinischen Doppelbindungen zugegeben werden und die Polymerisation in an sich bekannter Weise durch Zugabe einer radikalbildenden Verbindung gestartet wird.

Ein weiterer Gegenstand der Erfindung ist daher ein Verfahren zur Herstellung der erfindungsgemäßen Polymere, dadurch gekennzeichnet, dass
i) die Monomere, aus denen sich die Struktureinheiten der Formeln (1) und (2) ableiten und gegebenenfalls zusätzlich Monomere, aus denen sich die Struktureinheiten der Formel (3) oder der Formeln (3) und (4) ableiten, in einem protischen Lösungsmittel gelöst oder dispergiert werden,
ii) mit Ammoniak oder einer Li⁺-, Na⁺-, K⁺-, Ca⁺⁺-, Mg⁺⁺-, Zn⁺⁺- oder Al⁺⁺⁺ - enthaltenden Base, vorzugsweise der entsprechenden Hydroxide oder Carbonate, besonders bevorzugt der Hydroxide, und einem Alkylamin neutralisiert wird
iii) optional ein oder mehrere Vernetzer mit mindestens zwei olefinischen Doppelbindungen zugegeben werden und
iv) durch Zugabe einer radikalbildenden Verbindung die Polymerisation gestartet wird.

Für die erfindungsgemäße Anwendung ist es sehr wichtig, dass das oben genannte molare Verhältnis der Ionen ausgewählt aus NH₄⁺, Li⁺, Na⁺, K⁺, Ca⁺⁺, Mg⁺⁺, Zn⁺⁺ und Al⁺⁺⁺ zu den Alkylammonium-lonen von 97 : 3 bis 55 : 45 eingehalten wird und dass die kritischen Mizellbildungskonzentrationen (critical micelle concentration, CMC) der entsprechenden Hydrochlorid-Salze der eingesetzten Amine, d. h. der Ammoniumchloride XCI, unterhalb 15 g/l liegen.

Die erfindungsgemäßen Polymere haben ein hervorragendes Verdickungsvermögen, sowohl für Zusammensetzungen auf wässriger oder wässrig-alkoholischer als auch für Zusammensetzungen auf wässrig-tensidischer Basis, insbesondere aber für Zusammensetzungen mit einem hohen Ölanteil.

Die erfindungsgemäßen Polymere sind zudem in vorteilhafter Weise als Verdicker, Konsistenzgeber, Emulgator, Sensorikadditiv, Solubilisator, Dispergator, Gleitmittel, Haftmittel und Stabilisator geeignet.

Ein weiterer Gegenstand der Erfindung betrifft daher die Verwendung eines oder mehrerer der erfindungsgemäßen Polymere als Verdicker, Konsistenzgeber, Emulgator, Sensorikadditiv, Solubilisator, Dispergator, Gleitmittel, Haftmittel oder Stabilisator, vorzugsweise als Verdicker, Konsistenzgeber oder Stabilisator und besonders bevorzugt als Verdicker.

Die erfindungsgemäßen Polymere sind insbesondere als Verdicker, Konsistenzgeber, Emulgator, Sensorikadditiv, Solubilisator, Dispergator, Gleitmittel, Haftmittel und Stabilisator und vorzugsweise als Verdicker, Konsistenzgeber oder Stabilisator in Zusammensetzungen mit einem hohen Anteil an Ölkomponenten geeignet.

Die erfindungsgemäßen Polymere eignen sich außerordentlich bevorzugt zum Verdicken von Zusammensetzungen mit einem hohen Anteil an Ölkomponenten.

Eine bevorzugte Ausführungsform der Erfindung ist daher die Verwendung eines oder mehrerer der erfindungsgemäßen Polymere als Verdicker, Konsistenzgeber, Emulgator, Sensorikadditiv, Solubilisator, Dispergator, Gleitmittel, Haftmittel oder Stabilisator in Zusammensetzungen mit einem Ölanteil von > 5 Gew.-%, bevorzugt von 10 bis 60 Gew.-% und besonders bevorzugt von 20 bis 45 Gew.-%, bezogen auf die fertige Zusammensetzung, vorzugsweise als Verdicker, Konsistenzgeber oder Stabilisator und besonders bevorzugt als Verdicker.

Die Ölkörper können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren oder aus der Gruppe der Alkylbenzoate, bevorzugt C₁₂-C₁₅ Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkem-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318. Auch gehärtete Triglyceride sind bevorzugt. Auch Öle tierischen Ursprungs, beispielsweise Rindertalg, Perhydrosqualen, Lanolin können eingesetzt werden.

Eine weitere Klasse von bevorzugten Olkörpern sind die Benzoesäureester von linearen oder verzweigten C₈₋₂₂-Alkanolen, z. B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether, n-Hexyl-n-undecylether, Di-3-ethyldecylether, tert.-Butyl-n-octylether, iso-Pentyl-n-octylether, 2-Methyl-pentyl-n-octylether, Di-tert.-butylether und Di-isopentylether.

Ebenso in Betracht kommen verzweigte gesättigte oder ungesättigte Fettalkohole mit 6-30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol^{®} der EniChem, Augusta Industriale.

Eine weitere Klasse von bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat. Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®} S), Ozokerit, und Ceresin.

An Silikonölen bzw. -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Coming Corporation), erhältlichen Dimethicone, sowie die unter SilCare^{®} Silicone 41 M65, SilCare^{®} Silicone 41 M70, SilCare^{®} Silicone 41 M80 (Clariant) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SilCare^{®} Silicone 41M40, SilCare® Silicone 41 M50 (Clariant) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen Polymere zeichnen sich durch eine gute Hautmilde und ein angenehmes Hautgefühl aus und sind als Verdicker, Konsistenzgeber, Emulgator, Sensorikadditiv, Solubilisator, Dispergator, Gleitmittel, Haftmittel oder Stabilisator, vorzugsweise als Verdicker, Konsistenzgeber oder Stabilisator und besonders bevorzugt als Verdicker in kosmetischen, pharmazeutischen oder dermatologischen Zusammensetzungen geeignet.

Ein weiterer Gegenstand der Erfindung ist daher auch die Verwendung eines oder mehrerer der erfindungsgemäßen Polymere als Verdicker, Konsistenzgeber, Emulgator, Sensorikadditiv, Solubilisator, Dispergator, Gleitmittel, Haftmittel oder Stabilisator, vorzugsweise als Verdicker, Konsistenzgeber oder Stabilisator und besonders bevorzugt als Verdicker in kosmetischen, pharmazeutischen oder dermatologischen Zusammensetzungen. In einer bevorzugten Ausführungsform der Erfindung haben diese Zusammensetzungen einen Ölanteil von > 5 Gew.-%, bevorzugt von 10 bis 60 Gew.-% und besonders bevorzugt von 20 bis 45 Gew.-%, bezogen auf die fertige Zusammensetzung.

Weiterer Gegenstand der vorliegenden Erfindung sind kosmetische, pharmazeutische oder dermatologische Zusammensetzungen enthaltend ein oder mehrere erfindungsgemäße Polymere.

Die erfindungsgemäßen kosmetischen, pharmazeutischen und dermatologischen Zusammensetzungen enthalten die erfindungsgemäßen Polymere in Mengen von vorzugsweise 0,05 bis 10 Gew.-%, besonders bevorzugt von 0,1 bis 5 Gew.-% und insbesondere bevorzugt von 0,5 bis 2 Gew.%, bezogen auf die fertigen Zusammensetzungen.

Die erfindungsgemäßen kosmetischen, dermatologischen und pharmazeutischen Zusammensetzungen haben Viskositäten vorzugsweise im Bereich von 500 bis 300.000 mPa·s, besonders bevorzugt im Bereich von 1.000 bis 250.000 mPa·s, insbesondere bevorzugt im Bereich von 2.000 bis 150.000 mPa·s und außerordentlich bevorzugt im Bereich von 3.000 bis 100.000 mPa·s (25 °C, Brookfield RVT, T-C Spindel bei 5 rpm).

In einer weiteren bevorzugten Ausführungsform der Erfindung liegen die erfindungsgemäßen kosmetischen, dermatologischen oder pharmazeutischen Zusammensetzungen in Form von Fluids, Gelen, Ölen, Schäumen, Sprays, Lotionen oder Cremes vor.

Die erfindungsgemäßen kosmetischen, dermatologischen und pharmazeutischen Zusammensetzungen können auf wässriger, wässrig-alkoholischer Basis oder als Öl-in-Wasser Emulsion vorliegen.

In einer insbesondere bevorzugten Ausführungsform liegen die erfindungsgemäßen Zusammensetzungen als Emulsionen vom Typ Öl-in-Wasser bzw. Wasser-in-Öl, vorzugsweise als kosmetische, pharmazeutische oder dermatologische Emulsionen vom Typ Öl-in-Wasser, vor und enthalten bezogen auf das Gesamtgewicht der Zusammensetzung
a) bis zu 95 Gew.-%, vorzugsweise 60 bis 92 Gew.%, besonders bevorzugt 70 bis 90 Gew.-%, insbesondere bevorzugt 75 bis 85 Gew.-% einer Wasserphase,
b) bis zu 60 Gew.-%, vorzugsweise 0,1 bis 55 Gew.-%, besonders bevorzugt 1 bis 50 Gew.-%, insbesondere bevorzugt 3 bis 45 Gew.-% und außerordentlich bevorzugt 5 bis 40 Gew.-% einer Ölphase,
c) bis zu 15 Gew.-%, vorzugsweise 0,5 bis 12 Gew.-%, besonders bevorzugt 1 bis 8 Gew.-%, insbesondere bevorzugt 1 bis 5 Gew.-% eines oder mehrerer Emulgatoren und
d) 0,05 bis 10 Gew.-%, besonders bevorzugt 0,1 bis 5 Gew.-%, insbesondere bevorzugt 0,5 bis 2 Gew.-% an einem oder mehreren der erfindungsgemäßen Polymere.

Weiterhin vorteilhaft ist, dass die erfindungsgemäßen Polymere auch ohne Mitverwendung eines zusätzlichen Co-Emulgators und/oder ohne Mitverwendung eines zusätzlichen Konsistenzgebers eingesetzt werden können. Die Mitverwendung von Co-Emulgatoren und/oder Konsistenzgebern ist daher nicht zwingend, aber möglich. Eine Kombination mit anderen bekannten Co-Emulgatoren und/oder Konsistenzgebem kann zur Einstellung spezieller kosmetischer Profile und zur Ausnutzung synergistischer Effekte wünschenswert sein.

Weiterhin vorteilhaft ist, dass die erfindungsgemäßen Polymere auch ohne Mitverwendung eines Emulgators oder Tensids eingesetzt werden können, insbesondere in Öl-in-Wasser-Emulsionen. In einer insbesondere bevorzugten Ausführungsform der Erfindung liegen daher die erfindungsgemäßen Zusammensetzungen als Emulsionen vom Typ Öl-in-Wasser vor und enthalten keine Emulgatoren oder Tenside, d.h. sie liegen als Emulsionen vom Typ Öl-in-Wasser ohne Zusatz eines Emulgators oder Tensids vor.

In einer weiteren insbesondere bevorzugten Ausführungsform der Erfindung liegen die erfindungsgemäßen Zusammensetzungen als Gelcremes vom Typ Öl-in-Wasser, vorzugsweise als kosmetische, pharmazeutische oder dermatologische Gelcremes vom Typ Öl-in-Wasser, vor und enthalten bezogen auf das Gesamtgewicht der Zusammensetzung
a) bis zu 95 Gew.-%, vorzugsweise 50 bis 95 Gew.-%, besonders bevorzugt 70 bis 90 Gew.-%, insbesondere bevorzugt 75 bis 85 Gew.-% einer Wasserphase,
b) bis zu 60 Gew.-%, vorzugsweise 1 bis 30 Gew.-%, besonders bevorzugt 3 bis 25 Gew.-%, insbesondere bevorzugt 5 bis 15 Gew.-% einer Ölphase,
c) 0,05 bis 10 Gew.-%, besonders bevorzugt 0,1 bis 5 Gew.-%, insbesondere bevorzugt 0,5 bis 2 Gew.-% an einem oder mehreren der erfindungsgemäßen Polymere.

Für die erfindungsgemäßen Zusammensetzungen auf wässrig-alkoholischer oder alkoholischer Basis kommen alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol oder Glycerin sowie Alkylenglykole, insbesondere Propylen-, Butylen- oder Hexylenglykol, und Mischungen aus den genannten Alkoholen. Weitere bevorzugte Alkohole sind Polyethylenglykole mit einer relativen Molekülmasse unter 2.000. Insbesondere ist ein Einsatz von Polyethylenglykol mit einer relativen Molekülmasse zwischen 200 und 600 und von Polyethylenglykol mit einer relativen Molekülmasse zwischen 400 und 600 bevorzugt.

Die erfindungsgemäßen Polymere sind säurestabil und eignen sich zur Verwendung in kosmetischen, pharmazeutischen und/oder dermatologischen Zusammensetzungen mit niedrigem pH-Wert, insbesondere für die Pflege und Behandlung der Körper- oder Gesichtshaut.

Sei einigen Jahren hat sich der Einsatz von sauren Additiven in kosmetischen oder dermatologischen Zusammensetzungen etabliert. Insbesondere von Anti-Ageing Produkten ist die Abschilferung und Erneuerung der obersten Hautschichten des Stratum Corneums angestrebt. Für dieses sanfte peeling werden Hydroxysäuren und Ketosäuren, insbesondere Alpha Hydroxy Acids (AHA's) und Beta Hydroxy Acids eingesezt, welche linear, verzweigt oder cyclisch, gesättigt oder ungesättigt sein können. Die moderne Kosmetologie beschäftigt sich seit Jahren mit dieser Kategorie chemischer Verbindungen. Dazu zählen Glykolsäure aus Zuckerrohr, Milchsäure aus Sauermilch, Zitronensäure aus Zitrusfrüchten, Weinsäure aus Wein Salicylsäure, Brenztraubensäure aus Papayafrüchten.

Die Verwendung von sauren Additiven und deren Salze macht es teilweise notwendig, den pH-Wert der kosmetischen oder dermatologischen Zusammensetzungen in einen deutlich sauren Bereich einzustellen.

Die erfindungsgemäßen Zusammensetzungen können ferner einen oder mehrere saure organische Wirkstoffe enthalten, die Einsatzkonzentration ist typischerweise im Bereich von 0,01 bis 20 Gew.-%, bevorzugt im Bereich von 0,1 bis 5 Gew.-%. Als Wirkstoff in Betracht kommen Glykolsäure, Milchsäure, Zitronensäure, Weinsäure, Mandelsäure, Salicylsäure und alkylierte Salicylsäuren, Caffeic acid, Ascorbinsäure, Brenztraubensäure, Oligooxa Mono- und Dicarbonsäuren, Fumarsäure, Retinoesäure, Sulfonsäuren, Benzoesäure, Kojisäure, Fruchtsäure, Äpfelsäure, Gluconsäure, Pyruvic Acid, Galacturonic Acid, Ribonic Acid, und all deren Derivate, Polyglykoldisäuren in freier oder teilweiser neutralisierter Form, Vitamin C, Vitamin C-Derivate, Dihydroxyaceton oder Skin-whitening Actives wie Arbutin oder Glycyrrhetinsäure und deren Salze.
In einer weiteren bevorzugten Ausführungsform der Erfindung besitzen die erfindungsgemäßen Zusammensetzungen einen pH-Wert von 2 bis 6,5, vorzugsweise von 2 bis 6 und besonders bevorzugt von 3 bis 6.

Ein weiterer anwendungsrelevanter Vorteil der erfindungsgemäßen Polymere ist deren hervorragende Verdickerleistung und Stabilität auch in Gegenwart von Elektrolyten. Sie eignen sich zur Viskositätseinstellung von Zusammensetzungen mit hohem Elektrolytanteil und ergeben vorzugsweise klare Lösungen.

Als Elektrolyt zum Einsatz kommen anorganische Salze, bevorzugt Ammonium- oder Metallsalze, besonders bevorzugt von Halogeniden, beispielsweise CaCl₂, MgCl₂, LiCl, KCI, NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure oder Galacturonsäure.

Als Elektrolyt können die erfindungsgemäßen Zusammensetzungen auch Mischungen verschiedener Salze enthalten.

In einer weiteren bevorzugten Ausführungsform der Erfindung enthalten die erfindungsgemäßen Zusammensetzungen daher einen oder mehrere Elektrolyte.

Hierzu zählen auch wässrige Antiperspirant-Formulierungen enthaltend Aluminiumsalze, bevorzugt Aluminiumchlorohydrat oder Aluminium-Zirconium-Komplexsalze.

Der Gehalt an dem einen oder den mehreren Elektrolyten in den erfindungsgemäßen Zusammensetzungen, ist, bezogen auf die gesamte erfindungsgemäße Zusammensetzung, vorzugsweise von 0,1 bis 20,0 Gew.-%, besonders bevorzugt von 0,2 bis 10,0 Gew.-% und insbesondere bevorzugt von 0,5 bis 5,0 Gew.-%.

Die erfindungsgemäßen Polymere sind kompatibel mit organischen Komponenten und sind ideal geeignet zum Verdicken, Emulgieren und Stabilisieren von Sonnenschutzformulierungen. Überraschenderweise wurde festgestellt, dass die erfindungsgemäßen Polymere insbesondere Öl-in-Wasser Sonnenschutzformulierungen stabilisieren und zudem verbesserte Sonnenschutzfaktoren (SPF) und verbesserte Wasserbeständigkeit bewirken.

In einer weiteren bevorzugten Ausführungsform der Erfindung liegen die erfindungsgemäßen Zusammensetzungen in Form von Sonnenschutzmitteln vor und enthalten einen oder mehrere Sonnenschutzfilter zum Schutz der Haare und der Haut vor UV-Strahlen.

An Sonnenschutzfilter in Betracht kommen 4-Aminobenzoesäure, 3-(4'-Trimethylammonium)-benzyliden-boran-2-on-methylsulfat, Camphor Benzalkonium Methosulfat, 3,3,5-Trimethyl-cyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethyl-hexylester, ethoxyliertes Ethyl-4-amino-benzoat, 4-Methoxy-zimtsäure-isoamylester, 2,4,6-Tris-[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-amino-carbonyl)phenylamino]-1,3,5-triazin-2,4-yl)dümino]bis-(benzoesäure-2-ethylhexylester), Benzophenon-3, Benzophenon-4 (Säure), 3-(4'-Methylbenzyliden)-D,L-Campher, 3-Benzyliden-Campher, Salicylsäure-2-ethylhexylester, 4-Dimethylaminobenzoesäure-2-ethylhexylester, Hydroxy-4-methoxy-benzophenon-5-sulfonsäure (Sulfisobenzonum) und das Natriumsalz, 4-Isopropylbenzylsalicylat, N,N,N-Trimethyl-4-(2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, lsopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-Methylbenzyliden)-D,L-campher (4-Methylbenzyliden Camphor), Benzyliden-camphor-sulfonsäure, Octocrylen, Polyacrylamidomethyl-Benzyliden-Camphor, 2-Ethylhexyl salicylat (Octyl Salicylat), 4-Dimethyl-aminobenzoesäureethyl-2-hexylester (octyl dimethyl PABA), PEG-25 PABA, 2-Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4-tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzimidazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silyl-isopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäure/ Diisopropylzimtsäureester, 2-Ethylhexyl-p-methoxyzimtsäure, 2-Hydroxy-4-methoxy benzophenon, 2-Hydroxy-4-methoxybenzophenon-5-sulfsäure und das Trihydrat, sowie 2-Hydroxy-4-methoxybenzophenon-5-sulfonat Natriumsalz und Phenyl-benzimidazol-sulfonsäure.

Die Menge der vorgenannten Sonnenschutzfilter (eine oder mehrere Verbindungen) in den erfindungsgemäßen Zusammensetzungen beträgt vorzugsweise von 0,001 bis 30 Gew.-%, besonders bevorzugt von 0,05 bis 20 Gew.-% und insbesondere bevorzugt von 1 bis 10 Gew.%, bezogen auf das Gesamtgewicht der fertigen Zusammensetzung.

Die erfindungsgemäßen kosmetischen, pharmazeutischen und dermatologischen Zusammensetzungen können als weitere Hilfs- und Zusatzstoffe Tenside, Emulgatoren, Wachse, kationische Polymere, Filmbildner, weitere Verdicker, Gelierungsmittel, Überfettungsmittel, Rückfetter, antimikrobielle Wirkstoffe, biogene Wirkstoffe, Adstringentien, deodorierende Mittel, Antioxidantien, Feuchthaltemittel, Lösemittel, Silikonverbindungen, Farbstoffe, Duftstoffe, Konservierungsmittel, Perlglanzmittel, Trübungsmittel und/oder wasserlösliche Silikone enthalten.

Als Tenside können anionische, kationische, nichtionische, ampholytische Tenside und/oder Betaintenside enthalten sein.

Die Gesamtmenge der in den erfindungsgemäßen kosmetischen, pharmazeutischen und dermatologischen Zusammensetzungen eingesetzten Tenside beträgt, bezogen auf die fertigen Zusammensetzungen, bevorzugt von 1 bis 70 Gew.-%, besonders bevorzugt von 5 bis 40 Gew.-% und insbesondere bevorzugt von 10 bis 35 Gew.-%.

Als anionische Tenside bevorzugt sind (C₁₀-C₂₂)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Die Menge der anionischen Tenside in den erfindungsgemäßen kosmetischen, pharmazeutischen und dermatologischen Zusammensetzungen beträgt bevorzugt von 2 bis 30 Gew.%, besonders bevorzugt von 5 bis 25 Gew.-% und insbesondere bevorzugt von 12 bis 22 Gew.%, bezogen auf die fertigen Zusammensetzungen.
Bevorzugte kationische Tenside sind quartäre Ammonium-Salze, wie Di-(C₈-C₂₂)-Alkyl-dimethylammoniumchlorid oder -bromid, vorzugsweise Di-(C₈-C₂₂)-Alkyl-dimethylammoniumchlorid oder -bromid; (C₈-C₂₂)-Alkyl-dimethylethylammoniumchlorid oder -bromid; (C₈-C₂₂)-Alkyl-trimethylammoniumchlorid oder-bromid, vorzugsweise Cetyltrimethylammoniumchlorid oder-bromid und (C₈-C₂₂)-Alkyl-trimethylammoniumchlorid oder -bromid; (C₁₀-C₂₄)-Alkyl-dimethylbenzyl-ammoniumchlorid oder -bromid, vorzugsweise (C₁₂-C₁₈)-Alkyl-dimethylbenzyl-ammoniumchlorid, (C₈-C₂₂)-Alkyl-dimethylhydroxyethylammoniumchlorid,- phosphat, -sulfat, -lactat, (C₈-C₂₂)-Alkylamidopropyltrimethylammoniumchlorid, -methosulfat, N,N-bis(2-C₈-C₂₂-Alkanoyl-oxyethyl)-dimethylammoniumchlorid, -methosulfat, N,N-bis(2-C₈-C₂₂-Alkanoyl-oxyethyl)hydroxyethyl-methyl-ammoniumchlorid, -methosulfat.

Die Menge der kationischen Tenside in den erfindungsgemäßen kosmetischen, pharmazeutischen und dermatologischen Zusammensetzungen beträgt bevorzugt von 0,1 bis 10 Gew.-%, besonders bevorzugt von 0,5 bis 7 Gew.-% und insbesondere bevorzugt von 1 bis 5 Gew.-%, bezogen auf die fertigen Zusammensetzungen.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Die Menge der nichtionischen Tenside in den erfindungsgemäßen kosmetischen, pharmazeutischen und dermatologischen Zusammensetzungen (z. B. im Falle von Rinse-off-Produkten) liegt bevorzugt im Bereich von 1 bis 20 Gew.-%, besonders bevorzugt von 2 bis 10 Gew.-% und insbesondere bevorzugt von 3 bis 7 Gew.-%, bezogen auf die fertigen Zusammensetzungen.

Weiterhin können die erfindungsgemäßen kosmetischen, pharmazeutischen und dermatologischen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈- Alkyldimethylaminoxide, Fettsäureamidoalkyl-dimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die C₁₂- bis C₁₈-Dimethylaminohexanoate bzw. die C₁₀- bis C₁₈-Acylamidopropandimetylbetaine.

Die Menge der amphoteren Tenside und/oder Betaintenside in den erfindungsgemäßen kosmetischen, pharmazeutischen und dermatologischen Zusammensetzungen beträgt bevorzugt von 0,5 bis 20 Gew.-% und besonders bevorzugt von 1 bis 10 Gew.-%, bezogen auf die fertigen Zusammensetzungen.

Bevorzugte Tenside sind Laurylsulfat, Laurethsulfat, Cocoamidopropylbetain, Alkylbetaine wie z. B. Coco-Betain, Natriumcocoylglutamat und Lauroamphoacetat.

In einer weiteren bevorzugten Ausführungsform der Erfindung enthalten die erfindungsgemäßen kosmetischen, pharmazeutischen und dermatologischen Zusammensetzungen zusätzlich noch als schaumverstärkende Mittel Co-Tenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Erfindungsgemäße als Emulsionen vorliegende kosmetische, pharmazeutische und dermatologische Zusammensetzungen können ohne weiteren Emulgator erzeugt werden oder auch einen oder mehrere Emulgatoren enthalten. Diese Emulgatoren können gewählt werden aus der Gruppe der nichtionischen, anionischen, kationischen oder amphoteren Emulgatoren.

Als nichtionogene Emulgatoren kommen vorzugsweise in Betracht: Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole, Cetylstearylalkohole.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der
Gruppe Polyethylenglykol(20)glyceryllaurat,
Polyethylenglykol(6)glycerylcaprat/caprinat, Polyethylenglykol(20)glyceryloleat,
Polyethylenglykol(20)glycerylisostearat und
Polyethylenglykol(18)glyceryloleat/cocoat zu wählen.

Unter den Sorbitanestern eignen sich besonders
Polyethylenglykol(20)sorbitanmonolaurat,
Polyethylenglykol(20)sorbitanmonostearat,
Polyethylenglykol(20)sorbitanmonoisostearat,
Polyethylenglykol(20)sorbitanmonopalmitat,
Polyethylenglykol(20)sorbitanmonooleat.

Besonders vorteilhafte Emulgatoren sind Glycerylmonostearat, Glycerylmonooleat, Diglycerylmonostearat, Glycerylisostearat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglycol(2)stearylether (Steareth-2), Alkylmethiconcopolyole und Alkyl-Dimethiconcopolyole, insbesondere Cetyldimethiconcopolyol, Laurylmethiconcopolyol.

Die erfindungsgemäßen kosmetischen, pharmazeutischen und dermatologischen Zusammensetzungen können einen oder mehrere der Emulgatoren in Mengen von vorzugsweise 0,1 bis 20 Gew.-%, besonders bevorzugt von 1 bis 15 Gew.-% und insbesondere bevorzugt von 3 bis 10 Gew.-%, bezogen auf die fertigen Zusammensetzungen, enthalten.

Die erfindungsgemäßen kosmetischen, pharmazeutischen und dermatologischen Zusammensetzungen können Wachse, beispielsweise Paraffinwachse, Mikrowachse und Ozokerite, Bienenwachs und ihre Teilfraktionen sowie der Bienenwachsderivate, Wachse aus der Gruppe der homopolymeren Polyethylene oder Coplymere der α-Olefine, sowie natürliche Wachse wie Reiswachs, Candellilawachs, Carnaubawachs, Japanwachs oder Schellackwachs enthalten.

Als Stabilisatoren können Metallsalze von Fettsäuren, wie z. B. Magnesium-, Aluminium- und/oder Zinkstearat eingesetzt werden, bevorzugt in Mengen von 0,1 bis 10 Gew.-%, vorzugsweise von 0,5 bis 8 Gew.-% und besonders bevorzugt von 1 bis 5 Gew.%, bezogen auf die fertigen Zusammensetzungen.

Als kationische Polymere eignen sich die unter der INCI-Bezeichnung "Polyquatemium" bekannten, insbesondere Polyquaternium-31, Polyquatemium-16, Polyquaternium-24, Polyquaternium-7, Polyquatemium-22, Polyquaternium-39, Polyquatemium-28, Polyquaternium-2, Polyquatemium-10, Polyquatemium-11, sowie Polyquaternium 37&mineral oil&PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Die erfindungsgemäßen kosmetischen, pharmazeutischen und dermatologischen Zusammensetzungen können ein oder mehrere der oben genannten kationischen Polymere in Mengen von vorzugsweise 0,1 bis 5 Gew.-%, besonders bevorzugt von 0,2 bis 3 Gew.-% und insbesondere bevorzugt von 0,5 bis 2 Gew.-%, bezogen auf die fertigen Zusammensetzungen, enthalten.

Des Weiteren können die erfindungsgemäßen kosmetischen, pharmazeutischen und dermatologischen Zusammensetzungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester, Polyvinylalkohol, wasserlöslichen Acrylsäurepolymeren/Copolymeren bzw. deren Estern oder Salzen, beispielsweise Partialestercopolymere der Acryl/Methacrylsäure, wasserlöslicher Cellulose, beispielsweise Hydroxymethylcellulose, Hydroxyethylcellulose, Hydroxypropylcellulose, wasserlöslichen Quatemiums, Polyquatemiums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich.

Die erfindungsgemäßen kosmetischen, pharmazeutischen und dermatologischen Zusammensetzungen können einen oder mehrere Filmbildner in Mengen von vorzugsweise 0,1 bis 10 Gew.%, besonders bevorzugt von 0,2 bis 5 Gew.-% und insbesondere bevorzugt von 0,5 bis 3 Gew.-%, bezogen auf die fertigen Zusammensetzungen, enthalten.

Die gewünschte Viskosität der erfindungsgemäßen Zusammensetzungen kann durch Zugabe von weiteren Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer oder Natriumpolyacrylate Verwendung finden.

Bevorzugt enthalten die erfindungsgemäßen kosmetischen, pharmazeutischen und dermatologischen Zusammensetzungen 0,01 bis 20 Gew.%, besonders bevorzugt 0,1 bis 10 Gew.-%, insbesondere bevorzugt 0,2 bis 3 Gew.-% und ganz besonders bevorzugt 0,4 bis 2 Gew.-% an Verdickern bzw. Geliermitteln.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10 Gew.-%, besonders bevorzugt von 0,1 bis 5 Gew.-% und insbesondere bevorzugt von 0,5 bis 3 Gew.-% eingesetzt werden, bezogen auf die fertigen Zusammensetzungen.

An antimikrobiellen Wirkstoffen kommen Cetyltrimethylammoniumchlorid, Cetylpyridiniumchlorid, Benzethoniumchlorid, Diisobutylethoxyethyldimethylbenzylammoniumchlorid, Natrium-N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox^{®}, lodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen kosmetischen, pharmazeutischen und dermatologischen Zusammensetzungen enthalten die antimikrobiellen Wirkstoffe bevorzugt in Mengen von 0,001 bis 5 Gew.-%, besonders bevorzugt von 0,01 bis 3 Gew.-% und insbesondere bevorzugt von 0,1 bis 2 Gew.-%, bezogen auf die fertigen Zusammensetzungen.

Die erfindungsgemäßen kosmetischen, pharmazeutischen und dermatologischen Zusammensetzungen können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol^{®}, Allantoin, Phytantriol^{®}, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C-Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Die erfindungsgemäßen kosmetischen, dermatologischen oder pharmazeutischen Zusammensetzungen können biogene Wirkstoffe bevorzugt in Mengen von 0,001 bis 5 Gew.-%, besonders bevorzugt von 0,01 bis 3 Gew.-% und insbesondere bevorzugt von 0,1 bis 2 Gew.-%, bezogen auf die fertigen Zusammensetzungen, enthalten.

Die erfindungsgemäßen kosmetischen, pharmazeutischen und dermatologischen Zusammensetzungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50 Gew.-%, besonders bevorzugt in Mengen von 0,01 bis 10 Gew.-% und insbesondere bevorzugt in Mengen von 0,1 bis 10 Gew.-% enthalten. Als deodorierende Stoffe bevorzugt sind Allantoin und Bisabolol. Diese werden vorzugsweise in Mengen von 0,0001 bis 10 Gew.-% eingesetzt.

Die erfindungsgemäßen kosmetischen, pharmazeutischen und dermatologischen Zusammensetzungen können als Pigmente/Mikropigmente sowie als Sonnenschutzfilter mikrofeines Titandioxid, Glimmer-Titanoxid, Eisenoxide, Glimmer-Eisenoxid, Zinkoxid, Siliciumoxide, Ultramarinblau, Chromoxide enthalten.

Die erfindungsgemäßen kosmetischen, pharmazeutischen und dermatologischen Zusammensetzungen können Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie DL-Carnosin, D-Camosin, L-Camosin und deren Derivaten (z. B. Anserin), Carotinoiden, Carotinen (z. B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, trans-Stilbenoxid), Superoxid-Dismutase und den erfindungsgemäß geeigneten Derivaten (Salzen, Estern. Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen kosmetischen, pharmazeutischen und dermatologischen Zusammensetzungen beträgt vorzugsweise 0,001 bis 30 Gew.-%, besonders bevorzugt 0,05 bis 20 Gew.-% und insbesondere bevorzugt 1 bis 10 Gew.-%, bezogen auf das Gesamtgewicht der Zusammensetzung.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenol und dessen Derivaten, beispielsweise D-Panthenol (R-2,4-Dihydroxy-N-(3-hydroxypropyl)-3,3-dimethylbutamid), DL-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15 Gew.-% und besonders bevorzugt von 0,5 bis 5 Gew.-%, bezogen auf die fertigen Zusammensetzungen.

Weitere Zusatzstoffe können Siliconverbindungen sein, vorzugsweise Dimethylpolysiloxan, Methyl-phenylpolysiloxane, cyclische Silicone sowie amino-, fettsäure-, alkohol-, polyether-, epoxy-, fluor- und/oder alkylmodifizierte Siliconverbindungen, beispielsweise Alkylsilicone SilCare^{®} Silicone 41 M10, SilCare^{®} Silicone 41M15, SilCare^{®} Silicone 41 M20, SiICare^{®} Silicone 41 M30 (Clariant), Alkyltrimethicone SilCare^{®} 31 M30, SilCare^{®} 31 M40, SilCare^{®} 31 M50, SilCare^{®} 31 M60 (Clariant), Phenyltrimethicone SilCare^{®} 15M30, SilCare^{®} 15M40, SilCare^{®} 15M50, SilCare^{®} 15M60 (Clariant), Polyalkylarylsiloxane und Polyethersiloxan-Copolymere.

Die erfindungsgemäßen kosmetischen, pharmazeutischen und dermatologischen Zusammensetzungen können die oben genannten Siliconverbindungen vorzugsweise in Mengen von 0,1 bis 20 Gew.-%, besonders bevorzugt von 0,2 bis 15 Gew.-% und insbesondere bevorzugt von 0,5 bis 10 Gew.-% bezogen auf die fertigen Zusammensetzungen, enthalten.
Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Konservierungsmittel in Betracht kommen vorzugsweise Phenoxyethanol, Parabene, Pentandiol oder Sorbinsäure. Sie werden vorzugsweise in Mengen von 0,001 bis 5 Gew.-%, besonders bevorzugt von 0,01 bis 3 Gew.-%, insbesondere bevorzugt von 0,1 bis 2 Gew.%, bezogen auf die fertigen erfindungsgemäßen Zusammensetzungen, eingesetzt.

Als Säuren oder Laugen zur pH-Wert-Einstellung werden vorzugsweise Mineralsäuren, insbesondere HCl, anorganische Basen, insbesondere NaOH oder KOH, und organische Säuren, insbesondere Zitronensäure, verwendet.

In einer weiteren bevorzugten Ausführungsform der Erfindung besitzen die erfindungsgemäßen Zusammensetzungen einen pH-Wert im Bereich von 2 bis 12 und bevorzugt im Bereich von 3 bis 8.

Die Beschaffenheit der erfindungsgemäßen kosmetischen, pharmazeutischen und dermatologischen Zusammensetzungen ist ausgesprochen vorteilhaft: die Emulsionen sind cremig und salbig und haben überhaupt nicht das gelartige oder sogar gelatineartige Aussehen gewisser Emulsionen nach dem Stand der Technik, bei denen die äußere wässrige Phase verdickt ist.

Auch das kosmetische Gefühl auf der Haut ist sehr gut:
beim Auftragen verleiht die Emulsion ein Gefühl der Frische und des Komforts,
wobei sie gleichzeitig gehaltvoll und nährend wirkt; sie ist weich und komfortabel und in keiner Weise klebrig.

Die nachfolgenden Beispiele und Anwendungen sollen die Erfindung näher erläutern, ohne sie jedoch darauf zu beschränken (bei allen Prozentangaben handelt es sich um Gewichtsprozent; Gew.-%).

### Herstellbeispiel

Die Polymere 1 bis 13 und 15 bis 24 wurden analog der Herstellung von Polymer 14 hergestellt. Die Mengen der Ausgangsstoffe sind der Tabelle 1 für die vernetzten Polymere bzw. der Tabelle 2 für die unvernetzten Polymere zu entnehmen.

Herstellung von Polymer 14:
In einem 1.000 ml Kolben mit Ankerrührer, Rückflusskühler, Innenthermometer, Einleitungsmöglichkeit für N₂ und NH₃ wurden 490,5 g tert.-Butanol vorgelegt. Anschließend wurden 80,0 g 2-Acrylamido-2-methyl-propan-sulfonsäure eingetragen und unter starkem Rühren dispergiert, wobei eine Trübung des Lösungsmittels erhalten blieb. Anschließend wurden 14,0 g (C₂₀-C₂₂)-Alkyl-dimethylamin zugegeben. Über einen Zeitraum von 90 Minuten wurden 5,9 g Ammoniak in den überstehenden Gasraum eingeleitet und mindestens weitere 30 Minuten nachgerührt bis sich ein pH-Wert von 4 - 9 eingestellt hatte. Es wurden 4,22 g N-Vinylpyrrolidon und 1,6 g Trimethylolpropantriacrylat hinzugegeben und die Vorlage jeweils mit tert.-Butanol (ca. 6 ml) nachgespült, um Verluste bei der Zugabe zu minimieren. Das Reaktionsgemisch wurde dann auf eine Temperatur von 60 °C erwärmt, wobei die Reaktionsmischung durch gleichzeitiges Einleiten von N₂ inertisiert wurde. Nach Erreichen der Temperatur von 60 °C wurden 2,6 g Dilaurylperoxid zugegeben. Die Reaktion sprang unmittelbar nach Zugabe des Initiators an, was an einem Anstieg der Temperatur und am Ausflocken des Polymers zu erkennen war. Etwa 15 Minuten nach dem Einsetzen der Polymerisationsreaktion wurde die Stickstoffzufuhr abgestellt. Ungefähr 30 Minuten nach Zugabe des Starters erreichte die Temperatur ein Maximum (ca. 65 - 70 °C). Weitere 30 Minuten nach Durchlaufen dieses Maximums wurde zum Rückfluss erhitzt und unter diesen Bedingungen zwei Stunden nachgerührt. Der Inhalt des Reaktionsgefäßes nahm im Verlauf der Reaktion eine breiartige Konsistenz an, war aber noch gut rührbar. Anschließend wurde auf Raumtemperatur abgekühlt und der Feststoff abgesaugt. Die Paste wurde bei 60 - 70°C über 24 Stunden im Vakuumtrockenschrank getrocknet. Es wurden 92,2 g eines feinen weißen Pulvers erhalten.

Analog wurden Polymere hergestellt unter Variation des Alkylamins und des Verhältnisses Ammonium : Alkylammonium im Polymer.
Als Vergleich dienten zudem die Handelsprodukte Hostacerin^{®} AMPS (gemäß EP 0 816 403) und Aristoflex^{®} AVC (gemäß EP 1 116 733), beide mit einem Verhältnis Ammonium : Alkylammonium = 100 : 0 im Polymer.

**Tab. 1: Polymere 1 bis 18, vernetzt**

| Bsp. Nr. | Zusammensetzung | | | | | | |
|---|---|---|---|---|---|---|---|
| | Alkylamin | Alkylamin [g] | Alkylamin [Mol-%] | ATBS [g] | NVP [g] | TMPTA [g] | DLP [g] |
| Hostacerin^{®} AMPS EP 0816 403 | Ammoniak | | | | | | |
| Aristoflex^{®} AVC EP 1 116 733 | Ammoniak | | | | | | |
| 1 | Ethanolamin | 1,19 | 5,0 | 80,00 | 4,22 | 1,60 | 1,00 |
| 2 | Ethanolamin | 2,38 | 10,0 | 80,00 | 4,22 | 1,60 | 1,00 |
| 3 | Ethanolamin | 4,76 | 20,0 | 80,00 | 4,22 | 1,60 | 1,00 |
| 4 | Ethanolamin | 11,91 | 50,0 | 80,00 | 4,22 | 1,60 | 1,00 |
| 5 | Octylamin | 1,00 | 2,0 | 80,00 | 4,22 | 1,60 | 1,00 |
| 6 | Octylamin | 2,52 | 5,0 | 80,00 | 4,22 | 1,60 | 1,00 |
| 7 | Octylamin | 5,04 | 10,0 | 80,00 | 4,22 | 1,60 | 1,00 |
| 8 | SAPDMA | 2,87 | 2,0 | 80,00 | 4,22 | 1,60 | 1,00 |
| 9 | SAPDMA | 7,18 | 5,0 | 80,00 | 4,22 | 1,60 | 1,00 |
| 10 | SAPDMA | 14,35 | 10,0 | 80,00 | 4,22 | 1,60 | 1,00 |
| 11 | SAPDMA | 28,70 | 20,0 | 80,00 | 4,22 | 1,60 | 1,00 |
| 12 | SAPDMA | 71,76 | 50,0 | 80,00 | 4,22 | 1,60 | 1,00 |
| 13 | (C₂₀-C₂₂)-Alkyl- dimethylamin | 6,83 | 5,0 | 80,00 | 4,22 | 1,60 | 1,00 |
| 14 | (C₂₀-C₂₂)-Alkyl- dimethylamin | 14,00 | 10,0 | 80,00 | 4,22 | 1,60 | 1,00 |
| 15 | (C₂₀-C₂₂)-Alkyl-dimethylamin | 27,30 | 20,0 | 80,00 | 4,22 | 1,60 | 1,00 |
| 16 | (C₂₀-C₂₂)-Alkyl-dimethylamin | 68,25 | 50,0 | 80,00 | 4,22 | 1,60 | 1,00 |
| 17 | (C₂₀-C₂₂)-Alkyl-dimethylamin | 27,30 | 20,0 | 84,22 | - | 1,60 | 1,00 |
| 18 | (C₂₀-C₂₂)-Alkyl-dimethylamin | 68,25 | 10,0 | 84,22 | - | 1,60 | 1,00 |

**Tab. 2: Polymere 19 bis 24, unvernetzt**

| Bsp. Nr. | | Zusammensetzung | | | | | |
|---|---|---|---|---|---|---|---|
| | Alkylamin | Alkylamin [g] | Alkylamin [Mol-%] | ATBS [g] | NVP [g] | TMPTA [g] | DLP [g] |
| 19 | SAPDMA | 7,18 | 5,0 | 80,00 | 4,22 | - | 1,00 |
| 20 | SAPDMA | 14,35 | 10,0 | 80,00 | 4,22 | - | 1,00 |
| 21 | (C₂₀-C₂₂)-Alkyl-dimethylamin | 6,83 | 5,0 | 80,00 | 4,22 | - | 1,00 |
| 22 | (C₂₀-C₂₂)-Alkyl-dimethylamin | 14,00 | 10,0 | 80,00 | 4,22 | - | 1,00 |
| 23 | SAPDMA | 14,35 | 10,0 | 84,22 | - | - | 1,00 |
| 24 | (C₂₀-C₂₂)-Alkyl-dimethylamin | 6,83 | 5,0 | 84,22 | - | - | 1,00 |

| | | | | | | | |
|---|---|---|---|---|---|---|---|
| ATBS: 2-Acrylamido-2-methyl-propan-sulfonsäure NVP: N-Vinylpyrrolidon TMPTA: Trimethylolpropantriacrylat DLP: Dilaurylperoxid SAPDMA: Stearylamidopropyl dimethylamin ex Goldschmidt (C₂₀-C₂₂)-Alkyl-dimethylamin: Genamin^{®} 20/22 R302D - ex Clariant Octylamin: ex Aldrich Monoethanolamin: ex Merck | | | | | | | |

Die in den Tabellen 1 und 2 angegebenen Werte für Alkylamin in Mol-% sind auf die Gesamtmenge an Ammoniak und Alkylamin bezogen. Beispielsweise bedeutet 10,0 Mol-% Alkylamin für Polymer Nr. 14 in Tabelle 1, dass, bezogen auf die Gesamtmenge an Ammoniak und Alkylamin, 10,0 Mol-% Alkylamin und 90,0 Mol% Ammoniak zur Herstellung des Polymers verwendet worden sind.

Im Rahmen der vorliegenden Erfindung werden die CMC-Messungen nach ISO 4311 ("Anionic and non-ionic surface active agents - determination of the critical micellization concentration - method by measuring surface tension with a plate, stirrup or ring") durchgeführt. Wässrige Lösungen der Alkylamine werden dabei mit HCl auf einen pH 5,0 - 6,0 eingestellt. Es werden die CMC der korrespondierenden Hydrochloride auf einem KRÜSS plate Tensiometer gemessen.

Monoethanolamine hydrochloride:
Keine CMC, nicht grenzflächenaktiv
Stearylamidopropyldimethylamin (SAPDMA) hydrochlorid:
   CMC: 0,06 g/l
(C₂₀-C₂₂)-Alkyl-dimethylaminhydrochlorid:
   CMC: 0,12 g/l

Octylaminhydrochlorid:
CMC: 20,0 g/l

Im Folgenden wurden die erfindungsgemäßen Copolymere auf ihre Eignung als Verdicker und Konsistenzgeber geprüft. Als Testsystem mit hohem Ölanteil ohne Zusatz von Emulgator wurde folgende Zusammensetzung gewählt:

| | |
|---|---|
| 43 | % Paraffinöl |
| 0,8 | % Phenonip XB |

| | |
|---|---|
| 0,35 | % Polymer aus Tabelle 1 |
| 1 | % Glycerin |
| ad 100 | % Wasser |

Phenonip XB: Phenoxyethanol (und) Methylparaben (und) Ethylparaben (und) Propylparaben

**Tabelle 3: Viskositätsvermögen, Emulgiervermögen und Stabilitätsverhalten**

| Emulsion enthaltend Polymer Nr. | Viskosität 0,5 % in H₂O [mPa·s] | Viskosität 1,0 % in H₂O [mPa·s] | Emulgiervermögen | Viskosität Emulsion [mPa·s] | Stabilität -5 °C / +40 °C | Stabilität +50 °C |
|---|---|---|---|---|---|---|
| Hostacerin^{®} AMPS | 16.000 | 54.500 | nein | | | |
| Aristoflex^{®} AVC | 17.500 | 51.000 | nein | | | |
| 1 | 25.000 | 40.600 | nein | | | |
| 2 | 21.600 | 27.400 | nein | | | |
| 3 | 18.850 | 23.050 | nein | | | |
| 4 | 1.035 | 2.135 | nein | | | |
| 5 | 26.500 | 61.000 | nein | | | |
| 6 | 24.400 | 45.200 | nein | | | |
| 7 | 16.850 | 20.700 | nein | | | |
| 8 | 16.750 | 14.620 | nein | | | |
| 9 | 26.850 | 56.600 | ja | 4.000 | 7 Zyklen | 7 Wochen |
| 10 | 14.960 | 33.200 | ja | 4.500 | 7 Zyklen | 7 Wochen |
| 11 | 4.360 | 13.050 | ja | 3.500 | 14 Zyklen | 7 Wochen |
| 12 | Nicht löslich | | nein | | | |
| 13 | 16.100 | 51.200 | ja | 8.000 | 14 Zyklen | >7 Wochen |
| 14 | 9.060 | 32.800 | ja | 9.100 | 14 Zyklen | >7 Wochen |
| 15 | 2.280 | 14.300 | ja | 7.000 | 7 Zyklen | >7 Wochen |
| 16 | Nicht löslich | | nein | | | |
| 17 | 6.500 | 54.500 | ja | 9.000 | 7 Zyklen | >7 Wochen |
| 18 | 7.000 | 49.000 | ja | 10.500 | 14 Zyklen | >7 Wochen |

### Vergleichspolymere:

| | |
|---|---|
| Hostacerin^{®} AMPS | (INCI: Ammonium Polyacryldimethyltauramide) |
| Aristoflex^{®} AVC Copolymer) | (INCI: Ammonium Acryloyldimethyltaurate / VP |

Polymere 1-4 (Alkylamin nicht grenzflächenaktiv, CMC des Ethanolaminhydrochlorids >> 15 g/l)

Polymere 5-7 (CMC des Octylaminhydrochlorids > 15 g/l)

Polymer 8 (CMC des Alkylaminhydrochlorids < 15 g/l, aber molares Verhältnis von Ammonium : Alkylammonium größer als 97 : 3)

Polymere 12, 16 (CMC des Alkylaminhydrochlorids < 15 g/l, aber molares Verhältnis von Ammonium : Alkylammonium kleiner als 55 : 45)

### Erfindungsgemäße Polymere:

Polymere 9-11, 13-15, 17-24 (CMC des Alkylaminhydrochlorids < 15, molares Verhältnis von Ammonium : Alkylammonium zwischen 97 : 3 bis 55 : 45)

Die Ausprüfung der Polymere gemäß Tabelle 3 zeigte, dass die erfindungsgemäßen Polymere hervorragend geeignet sind als Verdicker und Konsistenzgeber von wässrigen Systemen, als Stabilisator von Emulsionen und Dispersionen, insbesondere von kosmetischen, dermatologischen und pharmazeutischen Zusammensetzungen mit hohem Ölanteil.

Insbesondere wurde überraschend gefunden, dass die erfindungsgemäßen Polymere Öl-in-Wasser Emulsionen mit hohem Ölanteil auch ohne Zugabe von Emulgatoren stabilisieren.

**Tab. 4: Lagerstabiltäten von Formulierungen I bis IV**

| Inhaltsstoffe | I | II | III | IV |
|---|---|---|---|---|
| Wasser, deionisiert | ad 100 % | ad 100 % | ad 100 % | ad 100 % |
| Glycerin | 20 | 3 | 1 | 1 |
| Niacinamid | 5 | 1 | 1 | 1 |
| Panthenol | 1 | 0,1 | 0,1 | 0,1 |
| Isohexadecan | 6 | 20 | 6 | - |
| Ethylparaben | 0,15 | 0,15 | 0,15 | 0,15 |
| Cocosöl | 4 | 4 | 4 | - |
| Petrolatum | 2 | 20 | 6 | 43 |
| Propylparaben | 0,1 | 0,1 | 0,1 | 0,1 |
| PEG-100 Stearat | 0,3 | 0,3 | 0,3 | - |
| Stearylalkohol | 0,6 | 0,6 | 0,6 | - |
| Cetylalkohol | 0,5 | 0,5 | 0,5 | - |
| Behenylalkohol | 0,4 | 0,4 | 0,4 | - |
| Tocopherolacetat | 0,5 | 0,5 | 0,5 | 0,5 |
| Stearinsäure | 0,1 | - | 0,1 | - |
| Polymer Bsp. 14 | 1 | 0,35 | - | 0,35 |
| Polymer Bsp. 10 | - | - | 0,35 | |
| Sorbitan Sesquiolate | - | - | 0,1 | 0,1 |
| NaOH | 0,011 | - | - | - |
| Dry Flo Plus | 2 | 2 | 2 | - |
| Benzylalkohol | 0,25 | 0,25 | 0,25 | 0,25 |
| Cyclopentasiloxane | 1,5 | 1,5 | 1,5 | - |
| Parfum | 0,3 | 0,3 | 0,3 | 0,3 |
| Viskosität [mPa·s] | 25000 | 35000 | 18000 | 30000 |
| pH-Wert | 5,5 | 5,5 | 5,5 | 5,5 |
| Stabilität (90 Tage, 45 °C) | stabil | stabil | stabil | stabil |
| Stabilität (-5 °C - 40 °C, 5 Zyklen ä 24 h) | stabil | stabil | stabil | stabil |

### Anwendungsbeispiele

### Beispiel 1: O/W - Hautmilch

| | | |
|---|---|---|
| A | Polymer Bsp. 15 | 0,50 % |
| | Isopropylpalmitat | 4,00 % |
| | Mandelöl | 4,00 % |
| | Weizenkeimöl | 1,00 % |
| | Cetiol^{®} SN (Henkel) | 8,00 % |
| | (Cetearylisononanoat) | |
| B | Aristoflex^{®} AVC (Clariant) | 0,30 % |
| | (Ammonium Acryloyldimethyltaurate/VP Copolymer) | |
| C | Wasser | ad 100 % |
| D | Duftstoffe | 0,30 % |

### Herstellung

| | |
|---|---|
| I | A und B mischen, dann C hinzugeben |
| II | D zu I hinzurühren |
| III | Emulsion homogenisieren |

Polymer Bsp. 15 wird als Emulgator eingesetzt und erhöht zudem die Konsistenz.

### Beispiel 2: O/W After-Sun-Milch

| | | |
|---|---|---|
| A | Polymer Bsp. 13 | 0,50 % |
| | Isopropylpalmitat | 15,00 % |
| | Cetiol^{®} SN (Henkel) | 15,00 % |
| | (Cetearylisononanoat) Sojaöl | 4,00 % |
| | Miglyol^{®} 812 (Dynamit Nobel) | 3,00 % |
| | (Capryl/Caprin Triglyceride) Jojobaöl | 3,00 % |
| | Weizenkeimöl | 1,00 % |
| B | AQUAMOLLIN^{®} BC Plv. hochkonz. (Clariant) Ethylendiamin Tetraacetat, Na-Salz | 0,10 % |
| | Zitronensäure (10 %ig) | 0,30 % |
| | Wasser | ad 100 % |
| | Glycerin | 3,00 % |
| | ALLANTOIN (Clariant) | 0,20 % |
| | (Allantoin) | |
| | Konservierungsmittel | q.s. |
| C | Ethanol | 1,50 % |
| | Parfümöl | 0.30 % |

### Herstellung

I Die Komponenten von A homogen verrühren II Bei ca. 35 °C B in I einrühren. Zum Schluss C zugeben III Die Emulsion homogenisieren

Polymer Bsp. 13 dient als Emulgator und Verdicker. Zusätzlich wird ein samtiges, weiches Hautgefühl erzeugt.

### Beispiel 3: W/O Creme

| | | |
|---|---|---|
| A | HOSTACERIN^{®} DGI (Clariant) | 4,00 % |
| | (Polyglyceryl-2 Sesquiisostearat) Bienenwachs | 2,00 % |
| | Polymer Bsp. 11 | 1,50 % |
| | Mineralöl, niedrig viskos | 5,00 % |
| | Vaseline | 10,00 % |
| | Cetiol^{®} V (Henkel KGaA) (Decyloleat) | 5,00 % |
| B | 1,2-Propylenglycol | 3,00 % |
| | Wasser | ad 100 % |
| | Konservierungsmittel | q.s. |
| C | Duftstoff | 0,40 % |

### Herstellung

| | |
|---|---|
| I | A bei 80 °C aufschmelzen |
| II | B auf 80 °C erhitzen |
| III | II in I einrühren |
| IV | Abkühlen unter Rühren |
| V | Bei 35 °C Zugabe von C zu IV |

Polymer Bsp. 11 wird als Konsistenzgeber und Stabilisator eingesetzt.

### Beispiel 4: Haargel mit konditionierenden Eigenschaften

| | | |
|---|---|---|
| A | Wasser | ad 100 % |
| | Panthenol | 1,50 % |
| | UVAsorb S5 (Benzophenone-4) | 0,05 % |
| | Farbstofflösung | q.s. |
| | Konservierungsmittel | q.s. |
| B | Emulsogen^{®} HCO 040 (Clariant) (PEG-40, hydriertes Rizinusöl) | 0,50 % |
| | Parfüm | q.s. |
| C | Polymer Bsp. 9 | 2,00 % |
| D | Gafquat 755N (ISP) | 2,50 % |
| | (Polyquatemium-11) | |

### Herstellung

- I: Komponenten A mischen
- II: Komponenten B mischen und zu I geben
- III: C zu D geben
- IV: III in II einrühren

Polymer Bsp. 9 wirkt als effizienter Verdicker mit zusätzlich konditionierenden Eigenschaften.

### Beispiel 9: Haargel mit starkem Halt

| | | |
|---|---|---|
| A | Wasser | ad 100 % |
| | PVP K-30 (ISP) | 4,00 % |
| | PVP | |
| | Ethanol | 30,00 % |
| | Panthenol | 0,50 % |
| | UVAsorb S5 | 0,05 % |
| | (Benzophenone-4) | |
| | Farbstofflösung | q.s. |
| | Konservierungsmittel | q.s. |
| B | Abil B 8851 (Goldschmidt) | 1,00 % |
| | (Dimethicon Copolyol) | |
| | Emulsogen^{®} HCO 040 (Clariant) | 0,50 % |
| | (PEG-40 hydriertes Rizinusöl) | |
| | Duftstoff | q.s. |
| C | Polymer Bsp. 15 | 2,50 % |

### Herstellung

| | |
|---|---|
| I | Komponenten A mischen |
| II | Komponenten B in I geben |
| III | Komponenten C in I geben |

Polymer Bsp. 15 wird als Verdicker mit sehr guter Alkoholtoleranz verwendet und dient zudem als Suspendiermittel und Stabilisator für die unlöslichen Ölanteile.

### Beispiel 10: O/W - Hautmilch mit keratolytischer Wirkung

| | | |
|---|---|---|
| A | Mineralöl | 4,00 % |
| | Mandelöl | 4,00 % |
| | Cetiol^{®} SN (Henkel) | 8,00 % |
| | (Cetearylisononanoat) | |
| | Cetylalkohol | 2,00 % |
| | Stearinsäure | 2,00 % |
| B | Polymer Bsp. 10 | 0,80 % |
| C | Wasser | ad 100 % |
| | Zitronensäure | 0,30 % |
| | Äpfelsäure | 0,40 % |
| | Glykolsäure | 0,70 % |
| | Milchsäure | 0,70 % |
| D | Duftstoffe | 0,30 % |

### Herstellung

| | |
|---|---|
| I | A und B mischen |
| II | Die Komponenten von C mischen |
| III | II zu I hinzugeben |
| IV | D zu I hinzurühren |
| V | Emulsion homogenisieren, pH 3,5 |

### Beispiel 11: O/W - Hautmilch für trockene Haut

| | | |
|---|---|---|
| A | Sorbitan Sesquioleat | 1,00 % |
| | Mineral Öl | 5,00 % |
| | Isopropyl Palmitat | 6,00 % |
| | Jojobaöl | 2,00 % |
| | Caprylic/Capric Triglycerid | 4,00 % |
| | Soyabohnenöl | 3,00 % |
| B | Polymer Bsp. 13 | 1 ,00 % |
| C | HOSTAPON^{®} CLG (Clariant) | 0,60 % |
| | (Natriumlauroylglutamat) | |
| | AQUAMOLLIN BC Plv. hochkonz. (Clariant) | 0,10 % |
| | (Ethylendiamin Tetraacetat, Na-Salz) | |
| | Zitronensäure (10 % wässrig) | 2,00 % |
| | Glycerin | 3,00 % |
| | Wasser | ad 100 % |
| | Konservierungsmittel | q.s. |
| D | Duftstoff | 0,30 % |

### Herstellung

| | |
|---|---|
| I | A und B mischen |
| II | Lösung von C in I einrühren |
| III | D zu II zufügen |
| IV | Emulsion homogenisieren |
| V | auf pH 4,8 einstellen |

### Beispiel 12: W/O Emulsion: Depigmentierungscreme

| | | |
|---|---|---|
| A | HOSTACERIN^{®} DGI (Clariant) | 4,00 % |
| | (Polyglyceryl-2 Sesquiisostearat) | |
| | Cetylalkohol | 1 ,20 % |
| | Stearinsäure | 1,00 % |
| | Cetiol^{®} V (Henkel KGaA) | 5,00 % |
| | (Decyloleat) | |
| | Bienenwachs | 2,00 % |
| | Cyclomethicon | 7,00 % |
| B | Polymer Bsp. 21 | 1,50 % |
| | Kojisäure | 1 ,00 % |
| | Kaffeesäure | 1,00 % |
| | Wasser | ad 100 % |
| C | PEG 600 (Clariant) | 10,00 % |
| | (PEG-12) | |
| | Konservierungsmittel | q.s. |
| D | Duftstoff | 0,40 % |

### Herstellung

| | |
|---|---|
| I | A bei 80 °C aufschmelzen |
| II | B in I einrühren |
| III | unter Rühren abkühlen |
| IV | bei 35°C C, dann D zu IV hinzufügen |
| V | pH 3,4 |

### Beispiel 13: Klares Duschbad mit guten Schaumeigenschaften

| | | |
|---|---|---|
| A | GENAPOL^{®} LRO flüssig (Clariant) | 40,00 % |
| | (Natriumlaurethsulfat) | |
| B | Duftstoff | 0,30 % |
| C | Wasser | ad 100 % |
| | Farbstoff | q.s. |
| | Konservierungsmittel | q.s. |
| | GENAGEN^{®} LDA (Clariant) | 6,00 % |
| | (Natriumlauroamphoacetat) | |
| | Zitronensäure | 5,00 % |
| | Ascorbinsäure | 0,50 % |
| D | Polymer Bsp. 18 | 1,50% |

### Herstellung

| | |
|---|---|
| I | B in A einrühren |
| II | Komponenten aus C nacheinander zu I zugeben |
| III | pH auf ca. 5,0 einstellen |
| IV | Einstellen der Viskosität durch Einrühren von D in III |

### Beispiel 14: Anti-age Gel

| | | |
|---|---|---|
| A | Glycerin | 3,00 % |
| | Wasser | ad 100 % |
| | Zitronensäure | 0,30 % |
| | Äpfelsäure | 0,40 % |
| | Glykolsäure | 0,70 % |
| | Milchsäure | 0,70 % |
| | Konservierungsmittel | q.s. |
| B | Duftstoff | 0,30 % |
| C | Polymer Bsp. 17 | 1,50 % |

### Herstellung

| | |
|---|---|
| I | A und B mischen |
| II | C zu I zufügen |
| III | pH auf 5,0 einstellen |

### Beispiel 15: Gel mit keratolytischer Wirkung

| | | |
|---|---|---|
| A | Wasser | ad 100 % |
| | Glycerin | 3,00 % |
| | 3, 6, 9-Trioxaundecanedioicsäure | 4,00 % |
| | Konservierungsmittel | q.s. |
| | Duftstoff | 0,30 % |
| | Polymer Bsp. 18 | 1 ,50 % |

### Herstellung

| | |
|---|---|
| I | Komponenten nacheinander vermischen |
| II | pH 3,8 einstellen |

### Beispiel 16: O/W - Hautmilch mit Thermalwasser

| | | |
|---|---|---|
| A | Polymer Bsp. 22 | 0,50 % |
| | Polymer Bsp. 24 | 1,00 % |
| B | Isopropylpalmitat | 4,00 % |
| | Mandelöl | 4,00 % |
| | Weizenkeimöl | 1,00 % |
| | Cetiol^{®} SN (Henkel) | 8,00 % |
| | (Cetearylisononanoat) | |
| C | Thermal-Wasser | ad 100 % |
| D | Duftstoffe | 0,30 % |
| | Konservierungsmittel | 0,70 % |

### Herstellung

| | |
|---|---|
| I | A in B unter Rühren dispergieren |
| II | C und D nacheinander zu I hinzufügen |
| III | Emulsion homogenisieren |

### Beispiel 17: O/W - Gesichtscreme

| | | |
|---|---|---|
| A | Polymer Bsp. 9 | 1,50 % |
| | Mineralöl | 5,00 % |
| | Cyclohexadimethylsiloxan | 5,00 % |
| B | Magnesiumascorbylphosphat | 0,30 % |
| | Wasser | ad 100 % |
| C | Duftstoffe | 0,30 % |
| | Konservierungsmittel | 0,70 % |

### Herstellung

| | |
|---|---|
| I | A unter Rühren dispergieren |
| II | B und C nacheinander zu I hinzufügen |
| III | Emulsion homogenisieren |

### Beispiel 18: body wash mit Totes Meer Salz

| | | |
|---|---|---|
| A | GENAPOL^{®} LRO flüssig (Clariant) | 40,00 % |
| | (Natriumlaurethsulfat) | |
| B | Duftstoff | 0,30 % |
| C | Wasser | ad 100 % |
| | Mischung von totem Meersalz | 10,00 % |
| D | Farbstoff | q.s. |
| | Konservierungsmittel | q.s. |
| | GENAGEN^{®} LDA (Clariant) | 6,00 % |
| | (Dinatriumlauroamphodiacetat) | |
| | Zitronensäure | q.s. |
| E | Polymer Bsp. 17 | 1,00 % |

### Herstellung

| | |
|---|---|
| I | B in A einrühren |
| II | Die Komponenten von C mischen und in I einrühren |
| III | Komponenten aus D nacheinander zu II zugeben |
| IV | Einstellen der Viskosität durch Einrühren von E in III |

### Beispiel 19: Sonnenschutzmilch,

| | | |
|---|---|---|
| A | Hostaphat^{®} CK 100 (Clariant) | 2,00 % |
| | (Kaliumcetylphosphat) | |
| | Mineralöl, niedrigviskos | 4,00 % |
| | Cetiol^{®} SN | 4,00 % |
| | (Cetearylilsononanoat) | |
| | Cetiol^{®} 868 | 4,00 % |
| | (Octylstearat) | |
| | Neo Heliopan^{®} E 1000 | 8,50 % |
| | (Isoamyl p-Methoxycinnamat) | |
| | Neo Heliopan^{®} BB | 8,50 % |
| | (Benzophenone-3) | |
| B | Polymer Bsp. 14 | 1,00 % |
| C | Wasser | ad 100 % |
| | Hostapon^{®} CCG (Clariant) | 0,60 % |
| | (Natriumcocoylglutamat) | |
| | Allantoin (Clariant) | 0,30 % |
| | Allantoin | |
| | Glycerin | 5,00 % |
| | Panthenol | 1,00 % |
| D | Duftstoff | 0,30 % |
| | Phenonip^{®} (Clariant) | 0,50 % |
| | Phenoxyethanol (und) Methylparaben (und) Butylparaben (und) Ethylparaben (und) Propylparaben | |

### Herstellung

| | |
|---|---|
| I | Schmelzen von A, dann Zugabe von B |
| II | Erwärmen von C auf 80 °C |
| III | Einrühren von II in I und Abkühlen unter Rühren |
| IV | Zugabe von D zu III bei 35 °C |
| V | Homogenisieren der Emulsion |

### Beispiel 20: Klares Antischuppenschampoo mit UV-Schutz

| | | |
|---|---|---|
| A | Octopirox^{®} (Clariant) | 0,50 % |
| | (Pirocton Olamin) | |
| B | Wasser | 10,00 % |
| | Polymer Bsp. 24 | 0,70 % |
| C | Genapol^{®} LRO flüssig (Clariant) | 30,00 % |
| | (Natriumlaurethsulfat) | |
| | Benzophenon-1 | 0,50 % |
| D | Duftstoff | 0,30 |
| E | Allantoin (Clariant) | 0,30 |
| F | Wasser | ad 100 % |
| G | Farbstofflösung | q.s. |
| | Panthenol | 1,00 % |
| | Extrapone Nettle Special | 2,00 % |
| | (Urtica Dioica (Nettle) Extract) | |
| | Genagen^{®} CAB (Clariant) | 8,00 % |

### Herstellung

| | |
|---|---|
| I | Mischen von A mit B |
| II | Zugabe von C zu I und Rühren bis zum Aufklaren der Lösung |
| III | Nacheinander Einrühren der Komponenten D in II |
| IV | Lösen von E in F unter Rühren und mildem Erwärmen und Zugabe zu III unter Rühren |
| V | Einrühren der Komponenten G in IV |
| VI | Einstellen des pH-Wertes |

## Patentansprüche

1. Polymer enthaltend
a) eine oder mehrere der wiederkehrenden Struktureinheiten der Formel (1) 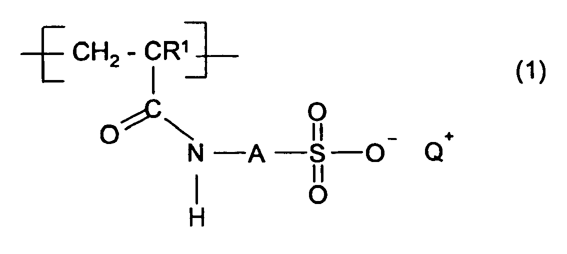 worin R¹ Wasserstoff, Methyl oder Ethyl und A C₁-C₈-Alkylen bedeutet, und Q⁺ für H⁺, NH₄⁺, Li⁺, Na⁺, K⁺, ½ Ca⁺⁺, ½ Mg⁺⁺, ½ Zn⁺⁺ oder ⅓ Al⁺⁺⁺ steht, und der Neutralisationsgrad der Struktureinheiten der Formel (1) von 50 bis 100 mol-%, vorzugsweise von 80 bis 100 mol-%, besonders bevorzugt von 90 bis 100 mol-% und insbesondere bevorzugt von 95 bis 100 mol-% beträgt,
und
b) eine oder mehrere der wiederkehrenden Struktureinheiten der Formel (2) 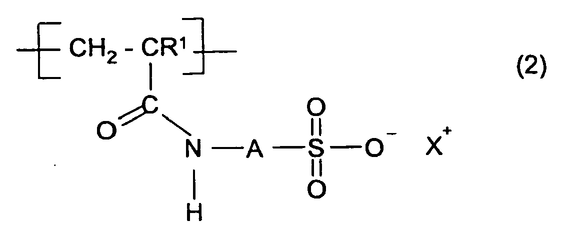 worin R¹ und A die Bedeutung von R¹ und A aus Formel (1) haben und X⁺ für [HNR⁵R⁶R⁷]⁺ steht, wobei R⁵, R⁶ und R⁷ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine C₆-C₂₂-Alkylamidopropylgruppe, eine lineare Mono-Hydroxyalkylgruppe mit 2 bis 10 Kohlenstoffatomen oder eine lineare oder verzweigte Di-Hydroxyalkylgruppe mit 3 bis 10 Kohlenstoffatomen sein können, und wobei mindestens einer der Reste R⁵, R⁶ und R⁷ nicht Wasserstoff ist, mit der Maßgabe, dass das molare Verhältnis der Struktureinheiten der Formel (1), worin Q⁺ NH₄⁺, Li⁺, Na⁺, K⁺, Ca⁺⁺, Mg⁺⁺, Zn⁺⁺ oder Al⁺⁺⁺ bedeutet, zu den Struktureinheiten der Formel (2) von 97 : 3 bis 55 : 45 beträgt, und die korrespondierenden Alkylammoniumchloride XCI eine kritische Mizellbildungskonzentration (critical micelle concentration, CMC) < 15 g/l besitzen,
und
d) 0 bis 8, vorzugsweise 0,01 bis 5 Gew.-% an vernetzenden Struktureinheiten, die aus Monomeren mit mindestens zwei olefinischen Doppelbindungen hervorgegangen sind.

2. Polymer nach Anspruch 1, **dadurch gekennzeichnet, dass** es
ab) 49,99 bis 98,99 Gew.-% einer Mischung der wiederkehrenden Struktureinheiten der Formeln (1) und (2),
c1) 1 bis 50 Gew.-% der wiederkehrenden Struktureinheit der Formel (3) 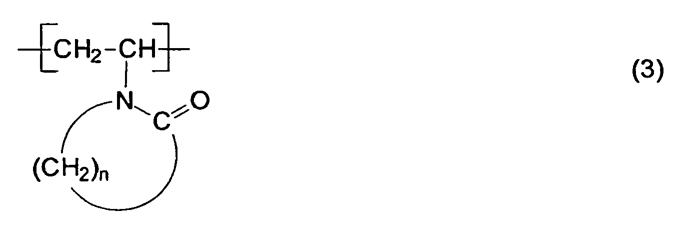 wobei n eine ganze Zahl von 2 bis 9 bedeutet,
oder
c2) 1 bis 50 Gew.-% einer Mischung der wiederkehrenden Struktureinheit der Formel (3) und der wiederkehrenden Struktureinheit der Formel (4) 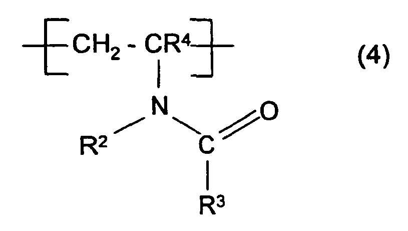 wobei R² und R³ gleich oder verschieden sein können und Wasserstoff oder eine lineare oder verzweigte Alkylgruppe mit 1 bis 22 Kohlenstoffatomen oder eine lineare oder verzweigte, einfach oder mehrfach ungesättigte Alkenylgruppe mit 2 bis 22 Kohlenstoffatomen bedeuten und R⁴ für Wasserstoff, Methyl oder Ethyl steht
und
d) 0 bis 8, vorzugsweise 0,01 bis 5, Gew.-% an vernetzenden Struktureinheiten, die aus Monomeren mit mindestens zwei olefinischen Doppelbindungen hervorgegangen sind,
enthält.

3. Polymer nach Anspruch 1, **dadurch gekennzeichnet, dass** es
ab) 92 bis 99,99 Gew.-% einer Mischung der wiederkehrenden Struktureinheiten der Formeln (1) und (2) und
d) 8 bis 0,01 Gew.-% an vernetzenden Struktureinheiten, die aus Monomeren mit mindestens zwei olefinischen Doppelbindungen hervorgegangen sind,
enthält.

4. Polymer nach Anspruch 1 oder 2, **dadurch gekennzeichnet, dass** es 69,5 bis 97,5, vorzugsweise 84,5 bis 96,5 Gew.-% einer Mischung der Struktureinheiten der Formeln (1) und (2), vorzugsweise abgeleitet von der 2-Acrylamido-2-methyl-propan-sulfonsäure, 2 bis 30, vorzugsweise 3 bis 15 Gew.-% der Struktureinheiten der Formel (3) oder einer Mischung der Struktureinheiten der Formeln (3) und (4), wobei die Struktureinheiten der Formel (3) vorzugsweise abgeleitet sind von N-Vinylpyrrolidon, und 0,2 bis 3, vorzugsweise 0,5 bis 2 Gew.-% an vernetzenden Struktureinheiten, die aus Monomeren mit mindestens zwei olefinischen Doppelbindungen hervorgegangen sind, enthält.

5. Polymer nach einem oder mehreren der Ansprüche 1 bis 4, **dadurch gekennzeichnet, dass** die Struktureinheiten der Formeln (1) und (2) abgeleitet sind von der 2-Acrylamido-2-methyl-propan-sulfonsäure.

6. Polymer nach einem oder mehreren der Ansprüche 2, 4 oder 5, **dadurch gekennzeichnet, dass** die Struktureinheiten der Formel (3) abgeleitet sind von N-Vinylpyrrolidon.

7. Polymer nach einem oder mehreren der Ansprüche 2 und 4 bis 6, **dadurch gekennzeichnet, dass** R² und R³ in den Struktureinheiten der Formel (4) Wasserstoff oder Methyl und R⁴ Wasserstoff ist.

8. Polymer nach einem oder mehreren der Ansprüche 1 bis 7, **dadurch gekennzeichnet, dass** das von H⁺ verschiedene Gegenion Q⁺ in den Struktureinheiten der Formel (1) ausgewählt ist aus NH₄⁺, Alkali⁺, wobei unter Alkali⁺ wiederum Na⁺ bevorzugt ist, und Erdalkali⁺⁺ und besonders bevorzugt NH₄⁺ ist.

9. Polymer nach einem oder mehreren der Ansprüche 1 bis 8, **dadurch gekennzeichnet, dass** das Kation X⁺ in den Struktureinheiten der Formel (2) ausgewählt ist aus Laurylamidopropyldimethylammonium,
Stearylamidopropyldimethylammonium, Behenylamidopropyldimethylammonium, C₁₂₋₁₈-Alkyldimethylammonium und C₂₀₋₂₂-Alkyldimethylammoniumion.

10. Verfahren zur Herstellung eines Polymers nach einem oder mehreren der Ansprüche 1 bis 9, **dadurch gekennzeichnet, dass**
i) Monomere, aus denen sich die Struktureinheiten der Formeln (1) und (2) ableiten und gegebenenfalls zusätzlich Monomere, aus denen sich die Struktureinheiten der Formel (3) oder der Formeln (3) und (4) ableiten, in einem protischen Lösungsmittel gelöst oder dispergiert werden,
ii) mit Ammoniak oder einer Li⁺-, Na⁺-, K⁺-, Ca⁺⁺-, Mg⁺⁺-, Zn⁺⁺- oder Al⁺⁺⁺-enthaltenden Base, vorzugsweise der entsprechenden Hydroxide oder Carbonate, besonders bevorzugt der Hydroxide, und einem Alkylamin neutralisiert wird,
iii) optional ein oder mehrere Vernetzer mit mindestens zwei olefinischen Doppelbindungen zugegeben werden und
iv) durch Zugabe einer radikalbildenden Verbindung die Polymerisation gestartet wird.

11. Verwendung eines oder mehrerer der Polymere nach einem oder mehreren der Ansprüche 1 bis 9 als Verdicker, Konsistenzgeber, Emulgator, Sensorikadditiv, Solubilisator, Dispergator, Gleitmittel, Haftmittel oder Stabilisator, vorzugsweise als Verdicker, Konsistenzgeber oder Stabilisator und besonders bevorzugt als Verdicker.

12. Verwendung nach Anspruch 11 in Zusammensetzungen mit einem Ölanteil von > 5 Gew.-%, bevorzugt von 10 bis 60 Gew.-% und besonders bevorzugt von 20 bis 45 Gew.-%, bezogen auf die fertige Zusammensetzung.

13. Verwendung nach Anspruch 11 oder 12 in kosmetischen, pharmazeutischen und dermatologischen Zusammensetzungen.

14. Kosmetische, pharmazeutische oder dermatologische Zusammensetzung enthaltend ein oder mehrere Polymere nach einem oder mehreren der Ansprüche 1 bis 9.

15. Zusammensetzung nach Anspruch 14, **dadurch gekennzeichnet, dass** sie in Form eines Fluids, Gels, Öls, Schaums, Sprays, einer Lotion oder Creme vorliegt.

16. Zusammensetzung nach Anspruch 14 oder 15, **dadurch gekennzeichnet, dass** sie als Emulsion vom Typ Öl-in-Wasser vorliegt und keine Emulgatoren oder Tenside enthält.

17. Zusammensetzung nach einem oder mehreren der Ansprüche 14 bis 16, **dadurch gekennzeichnet, dass** sie einen pH-Wert von 2 bis 6,5, vorzugsweise von 2 bis 6 und besonders bevorzugt von 3 bis 6, enthält.

18. Zusammensetzung nach einem oder mehreren der Ansprüche 14 bis 17, **dadurch gekennzeichnet, dass** sie einen oder mehrere Elektrolyte enthält.

19. Zusammensetzung nach einem oder mehreren der Ansprüche 14 bis 18, **dadurch gekennzeichnet, dass** sie in der Form eines Sonnenschutzmittels vorliegt und einen oder mehrere Sonnenschutzfilter zum Schutz der Haare und der Haut vor UV-Strahlen enthält.

## Claims

1. A polymer comprising
a) one or more of the structural repeat units of the formula (1) 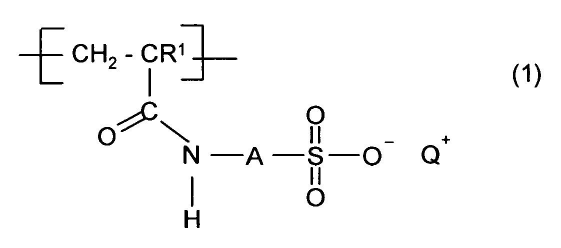 in which R¹ is hydrogen, methyl or ethyl and A is C₁-C₈-alkylene, and Q⁺ is H⁺, NH₄⁺, Li⁺, Na⁺, K⁺, ½ Ca⁺⁺, ½ Mg⁺⁺, ½ Zn⁺⁺ or ⅓ Al⁺⁺⁺, and the degree of neutralization of the structural units of the formula (1) is from 50 to 100 mol%, preferably from 80 to 100 mol%, more preferably from 90 to 100 mol%, and with more particular preference from 95 to 100 mol%,
and
b) one or more of the structural repeat units of the formula (2) 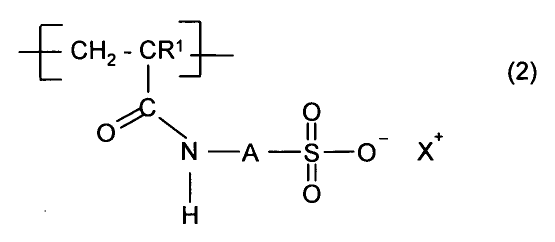 in which R¹ and A have the definition of R¹ and A from formula (1) and X⁺ is [HNR⁵R⁶R⁷]⁺, where R⁵, R⁶, and R⁷ independently of one another can be hydrogen, a linear or branched alkyl group having 1 to 22 carbon atoms, a linear or branched, singly or multiply unsaturated alkenyl group having 2 to 22 carbon atoms, a C₆-C₂₂-alkylamidopropyl group, a linear mono-hydroxyalkyl group having 2 to 10 carbon atoms or a linear or branched dihydroxyalkyl group having 3 to 10 carbon atoms, and where at least one of the radicals R⁵, R⁶, and R⁷ is not hydrogen, with the proviso that the molar ratio of the structural units of the formula (1) in which Q⁺ is NH₄⁺, Li⁺, Na⁺, K⁺, Ca⁺⁺, Mg⁺⁺, Zn⁺⁺ or Al⁺⁺⁺ to the structural units of the formula (2) is from 97:3 to 55:45, and the corresponding alkylammonium chlorides XCl possess a critical micelle concentration (CMC) < 15 g/l,
and
d) 0% to 8%, preferably 0.01% to 5%, by weight of crosslinking structural units originating from monomers having at least two olefinic double bonds.

2. The polymer as claimed in claim 1, **characterized in that** it comprises
ab) 49.99% to 98.99% by weight of a mixture of the structural repeat units of the formulae (1) and (2),
c1) 1% to 50% by weight of the structural repeat unit of the formula (3) 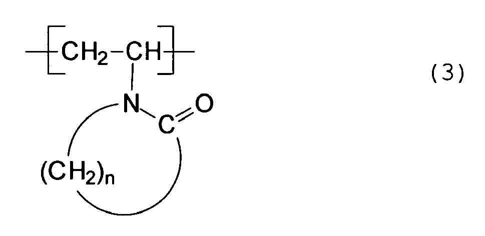 where n is an integer from 2 to 9,
or
c2) 1% to 50% by weight of a mixture of the structural repeat unit of the formula (3) and the structural repeat unit of the formula (4) 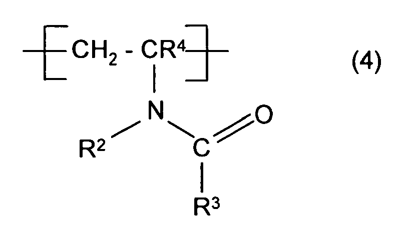 where R² and R³ can be alike or different and are hydrogen or a linear or branched alkyl group having 1 to 22 carbon atoms or a linear or branched, singly or multiply unsaturated alkenyl group having 2 to 22 carbon atoms, and R⁴ is hydrogen, methyl or ethyl,
and
d) 0% to 8%, preferably 0.01% to 5%, by weight of crosslinking structural units originating from monomers having at least two olefinic double bonds.

3. The polymer as claimed in claim 1, **characterized in that** it comprises
ab) 92% to 99.99% by weight of a mixture of the structural repeat units of the formulae (1) and (2) and
d) 8% to 0.01% by weight of crosslinking structural units originating from monomers having at least two olefinic double bonds.

4. The polymer as claimed in claim 1 or 2, **characterized in that** it comprises
69.5% to 97.5%, preferably 84.5% to 96.5%, by weight of a mixture of the structural units of the formulae (1) and (2), preferably derived from 2-acrylamido-2-methylpropanesulfonic acid, 2% to 30%, preferably 3% to 15%, by weight of the structural units of the formula (3) or of a mixture of the structural units of the formulae (3) and (4), the structural units of the formula (3) being derived preferably fromN-vinylpyrrolidone, and
0.2% to 3%, preferably 0.5% to 2%, by weight of crosslinking structural units originating from monomers having at least two olefinic double bonds.

5. The polymer as claimed in one or more of claims 1 to 4, **characterized in that** the structural units of the formulae (1) and (2) are derived from 2-acrylamido-2-methylpropanesulfonic acid.

6. The polymer as claimed in one or more of claims 2, 4, and 5, **characterized in that** the structural units of the formula (3) are derived from N-vinylpyrrolidone.

7. The polymer as claimed in one or more of claims 2 and 4 to 6, **characterized in that** R² and R³ in the structural units of the formula (4) are hydrogen or methyl and R⁴ is hydrogen.

8. The polymer as claimed in one or more of claims 1 to 7, **characterized in that** the non-H⁺ counterion Q⁺ in the structural units of the formula (1) is selected from NH₄⁺, alkali metal⁺, with Na⁺ being preferred in turn among alkali metal⁺, and alkaline earth metal⁺⁺, and with particular preference is NH₄⁺.

9. The polymer as claimed in one or more of claims 1 to 8, **characterized in that** the cation X⁺ in the structural units of the formula (2) is selected from laurylamidopropyldimethylammonium, stearylamidopropyldimethylammonium, behenylamidopropyldimethylammonium, C₁₂₋₁₈-alkyldimethylammonium, and C₂₀₋₂₂-alkyldimethylammonium ion.

10. A process for preparing a polymer as claimed in one or more of claims 1 to 9, **characterized in that**
i) monomers from which the structural units of the formulae (1) and (2) derive, and additionally, if desired, monomers from which the structural units of the formula (3) or of the formulae (3) and (4) derive, are subjected to dispersion or dissolution in a protic solvent,
ii) neutralization is carried out with ammonia or with a base containing Li⁺, Na⁺, K⁺, Ca⁺⁺, Mg⁺⁺, Zn⁺⁺ or Al⁺⁺⁺, preferably of the corresponding hydroxides or carbonates, with particular preference the hydroxides, and with an alkylamine,
iii) optionally one or more crosslinkers having at least two olefinic double bonds are added, and
iv) the polymerization is initiated by adding a free-radical-forming compound.

11. The use of one or more polymers as claimed in one or more of claims 1 to 9 as a thickener, bodying agent, emulsifier, sensorial additive, solubilizer, dispersant, lubricant, adhesive or stabilizer, preferably as a thickener, bodying agent or stabilizer, and, with particular preference, as a thickener.

12. The use as claimed in claim 11 in compositions having an oil fraction of > 5% by weight, preferably of 10% to 60% by weight, and more preferably of 20% to 45% by weight, based on the completed composition.

13. The use as claimed in claim 11 or 12 in cosmetic, pharmaceutical, and dermatological compositions.

14. A cosmetic, pharmaceutical or dermatological composition comprising one or more polymers as claimed in one or more of claims 1 to 9.

15. The composition as claimed in claim 14, **characterized in that** it is in the form of a fluid, gel, oil, foam, spray, lotion or cream.

16. The composition as claimed in claim 14 or 15, **characterized in that** it is in the form of an oil-in-water emulsion containing no emulsifiers or surfactants.

17. The composition as claimed in one or more of claims 14 to 16, **characterized in that** it has a pH of 2 to 6.5, preferably of 2 to 6 and more preferably of 3 to 6.

18. The composition as claimed in one or more of claims 14 to 17, **characterized in that** it comprises one or more electrolytes.

19. The composition as claimed in one or more of claims 14 to 18, **characterized in that** it is in the form of a sunscreen composition and comprises one or more sun protection filters for protecting the hair and the skin from UV rays.

## Revendications

1. Polymère contenant
a) un ou plusieurs des motifs structuraux répétitifs
de formule (1) 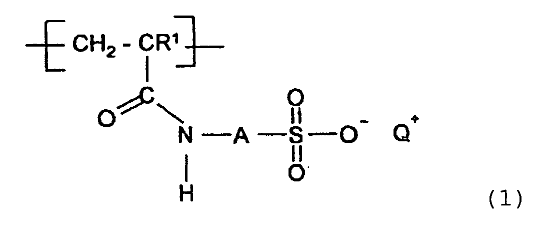 dans laquelle R¹ représente un atome d'hydrogène, le groupe méthyle ou éthyle et A représente un groupe alkylène en C₁-C₈, et Q⁺ représente H⁺, NH₄⁺, Li⁺, Na⁺, K⁺, ½ Ca⁺⁺, ½ Mg⁺⁺, ½ Zn⁺⁺ ou ⅓ Al⁺⁺⁺, et le degré de neutralisation des motifs structuraux de formule (1) vaut de 50 à 100 % en moles, de préférence de 80 à 100 % en moles, de façon particulièrement préférée de 90 à 100 % en moles et de façon tout particulièrement préférée de 95 à 100 % en moles,
et
b) un ou plusieurs des motifs structuraux répétitifs de formule (2) 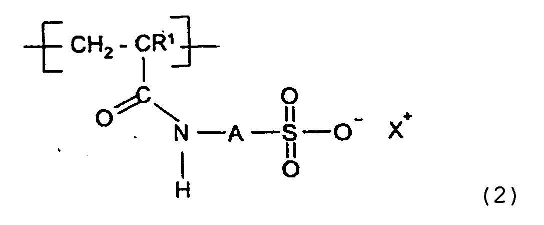 dans laquelle R¹ et A ont la signification de R¹ et A de la formule (1) et X⁺ représente [HNR⁵R⁶R⁷]⁺, R⁵ R⁶ et R⁷ pouvant être, chacun indépendamment, un atome d'hydrogène, un groupe alkyle linéaire ou ramifié ayant de 1 à 22 atomes de carbone, un groupe alcényle linéaire ou ramifié, une ou plusieurs fois insaturé, ayant de 2 à 22 atomes de carbone, un groupe alkyl(C₆-C₂₂)amidopropyle, un groupe mono-hydroxyalkyle linéaire ayant de 2 à 10 atomes de carbone ou un groupe di-hydroxyalkyle linéaire ou ramifié ayant de 3 à 10 atomes de carbone, et au moins l'un des radicaux R⁵, R⁶ et R⁷ n'étant pas un atome d'hydrogène, étant entendu que le rapport molaire des motifs structuraux de formule (1), dans lesquels Q⁺ représente H⁺, NH₄⁺, Li⁺, Na⁺, K⁺, Ca⁺⁺, Mg⁺⁺, Zn⁺⁺ ou Al⁺⁺⁺, aux motifs structuraux de formule (2) vaut de 97:3 à 55:45, et les chlorures d'alkylammonium XCl correspondants ont une concentration critique de formation de micelles (critical micelle concentration, CMC) < 15 g/l,
et
d) 0 à 8, de préférence 0,01 à 5 % en poids de motifs structuraux réticulants, qui sont issus de monomères comportant au moins deux doubles liaisons oléfiniques.

2. Polymère selon la revendication 1, **caractérisé en ce qu'**il contient
ab) 49,99 à 98,99 % en poids d'un mélange des motifs structuraux répétitifs de formules (1) et (2),
c1) 1 à 50 % en poids du motif structural répétitif de formule (3) 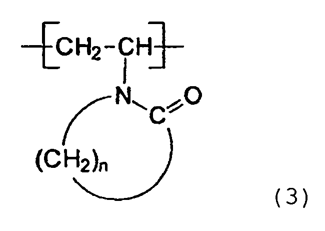 n représentant un nombre entier valant de 2 à 9,
ou
c2) 1 à 50 % en poids d'un mélange du motif structural répétitif de formule (3) et du motif structural répétitif de formule (4) 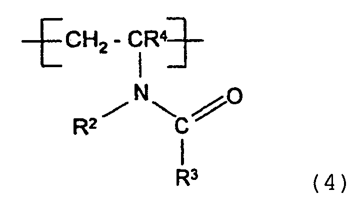 où R² et R³ peuvent être identiques ou différents et représentent un atome d'hydrogène ou un groupe alkyle linéaire ou ramifié ayant de 1 à 22 atomes de carbone ou un groupe alcényle linéaire ou ramifié, une ou plusieurs fois insaturé, ayant de 2 à 22 atomes de carbone et R⁴ représente un atome d'hydrogène, le groupe méthyle ou éthyle
et
d) 0 à 8, de préférence 0,01 à 5 % en poids de motifs structuraux réticulants qui sont issus de monomères comportant au moins deux doubles liaisons oléfiniques.

3. Polymère selon la revendication 1, **caractérisé en ce qu'**il contient
ab) 92 à 99,99 % en poids d'un mélange des motifs structuraux répétitifs de formules (1) et (2) et
d) 8 à 0,01 % en poids de motifs structuraux réticulants qui sont issus de monomères comportant au moins deux doubles liaisons oléfiniques.

4. Polymère selon la revendication 1 ou 2, **caractérisé en ce qu'**il contient 69,5 à 97,5, de préférence 84,5 à 96,5% en poids d'un mélange des motifs structuraux de formules (1) et (2), de préférence dérivés de l'acide 2-acrylamido-2-méthyl-propane-sulfonique, 2 à 30, de préférence 3 à 15 % en poids des motifs structuraux de formule (3) ou d'un mélange des motifs structuraux de formules (3) et (4), les motifs structuraux de formule (3) étant de préférence dérivés de N-vinylpyrrolidone, et 0,2 à 3, de préférence 0,5 à 2 % en poids de motifs structuraux réticulants qui sont issus de monomères comportant au moins deux doubles liaisons oléfiniques.

5. Polymère selon une ou plusieurs des revendications 1 à 4, **caractérisés en ce que** les motifs structuraux de formules (1) et (2) sont dérivés de l'acide 2-acrylamido-2-méthyl-propane-sulfonique.

6. Polymère selon une ou plusieurs des revendications 2, 4 ou 5, **caractérisé en ce que** les motifs structuraux de formule (3) sont dérivés de N-vinylpyrrolidone.

7. Polymère selon une ou plusieurs des revendications 2 et 4 à 6, **caractérisé en ce que** R² et R³ dans les motifs structuraux de formule (4) représentent un atome d'hydrogène ou le groupe méthyle et R⁴ est un atome d'hydrogène.

8. Polymère selon une ou plusieurs des revendications 1 à 7, **caractérisé en ce que** l'ion opposé Q⁺ différent de H⁺ dans les motifs structuraux de formule (1) est choisi parmi NH₄⁺, un métal alcalin⁺, parmi les alcalins⁺ à son tour Na⁺ étant préféré, et un métal alcalino-terreux⁺⁺, et de façon particulièrement préférée est NH₄⁺.

9. Polymère selon une ou plusieurs des revendications 1 à 8, **caractérisés en ce que** le cation X⁺ dans les motifs structuraux de formule (2) est choisi parmi les ions laurylamidopropyldiméthylammonium, stéarylamidopropyldiméthylammonium, béhénylamidopropyldiméthylammonium, alkyl(C₁₂-C₁₈)diméthylammonium et alkyl(C₂₀-C₂₂)diméthylammonium.

10. Procédé pour la préparation d'un polymère selon une ou plusieurs des revendications 1 à 9, **caractérisé en ce que**
i) on disperse ou dissout dans un solvant protique des monomères desquels dérivent les motifs structuraux de formules (1) et (2) et éventuellement en outre des monomères supplémentaires desquels dérivent les motifs structuraux de formule (3) ou de formules (3) et (4),
ii) on neutralise avec de l'ammoniac ou une base contenant Li⁺, Na⁺, K⁺, Ca⁺⁺, Mg⁺⁺, Zn⁺⁺ ou Al⁺⁺⁺, de préférence les hydroxydes ou carbonates correspondants, de façon particulièrement préférée les hydroxydes, et une alkylamine,
iii) en option on ajoute un ou plusieurs agents de réticulation comportant au moins deux doubles liaisons oléfiniques et
iv) on déclenche la polymérisation par addition d'un composé générateur de radicaux.

11. Utilisation d'un ou plusieurs des polymères selon une ou plusieurs des revendications 1 à 9, en tant qu'épaississant, agent de consistance, émulsifiant, additif sensoriel, solubilisant, dispersant, lubrifiant, adhésif ou stabilisant, de préférence en tant qu'épaississant, agent de consistance ou stabilisant et en particulier en tant qu'épaississant.

12. Utilisation selon la revendication 11, dans des compositions ayant une teneur en huile > 5 % en poids, de préférence de 10 à 60 % en poids et de façon particulièrement préférée de 20 à 45 % en poids, par rapport à la composition finale.

13. Utilisation selon la revendication 11 ou 12, dans des compositions cosmétiques, pharmaceutiques et dermatologiques.

14. Composition cosmétique, pharmaceutique ou dermatologique, contenant un ou plusieurs polymères selon une ou plusieurs des revendications 1 à 9.

15. Composition selon la revendication 14, **caractérisée en ce qu'**elle se trouve sous forme d'un fluide, d'un gel, d'une huile, d'une mousse, d'une composition à pulvériser en aérosol, d'une lotion ou d'une crème.

16. Composition selon la revendication 14 ou 15, **caractérisée en ce qu'**elle se trouve sous forme d'émulsion du type huile-dans-eau et ne contient pas d'émulsifiants ni de tensioactifs.

17. Composition selon une ou plusieurs des revendications 14 à 16, **caractérisée en ce qu'**elle présente un pH de 2 à 6,5, de préférence de 2 à 6 et de façon particulièrement préférée de 3 à 6.

18. Composition selon une ou plusieurs des revendications 14 à 17, **caractérisée en ce qu'**elle contient un ou plusieurs électrolytes.

19. Composition selon une ou plusieurs des revendications 14 à 18, **caractérisée en ce qu'**elle se trouve sous forme d'un produit antisolaire et contient un ou plusieurs filtres antisolaires pour la protection des cheveux et de la peau contre les rayons UV.
